# EUROPEAN PATENT APPLICATION

(11) **EP 4 600 331 A1**
(43) Date of publication of application: **13.08.2025**
(21) Application number: 23874948.5
(22) Date of filing: 05.10.2023
(51) Int. Cl.: C10G 2/00, C07C 1/04, C07C 11/06, C07C 15/02, C10L 3/12

(54) **USEFUL HYDROCARBON PRODUCTION METHOD AND USEFUL HYDROCARBON PRODUCTION APPARATUS**

(30) Priority: 05.10.2022 JP 2022160892
(71) Applicant: FURUKAWA ELECTRIC CO., LTD., Tokyo 100-8322 (JP)
(72) Inventor: KAWAMATA, Yuki, Tokyo 100-8322 (JP); IWANO, Yuki, Tokyo 100-8322 (JP); TAKAHASHI, Hiroko, Tokyo 100-8322 (JP); BAMBA, Yuichiro, Tokyo 100-8322 (JP); HIRANO, Junya, Tokyo 100-8322 (JP); FUJIKAWA, Takashi, Tokyo 100-8322 (JP); LEE, Yu, Tokyo 100-8322 (JP); YAMANAKA, Nobumitsu, Tokyo 100-8322 (JP)
(74) Representative: Papula Oy
(86) International application number: PCT/JP2023/036449
(87) International publication number: WO 2024/075823

(57) **Abstract**

Provided is a useful hydrocarbon production method comprising: a methanation step for generating a first gas containing methane from a mixed gas containing hydrogen and carbon dioxide; a dry reforming step for generating a second gas containing carbon monoxide and hydrogen from the first gas; a useful hydrocarbon generating step for generating a third gas containing a useful hydrocarbon from the carbon monoxide and hydrogen in the second gas; and a recycling step for separating a recycle gas for dry reforming, which contains at least carbon dioxide from the third gas and supply same to the dry reforming step.

## Description

### TECHNICAL FIELD

The present disclosure relates to a useful hydrocarbon production method and a useful hydrocarbon production device.

### BACKGROUND ART

Useful hydrocarbons such as various lower olefins and aromatic hydrocarbons which are basic chemical raw materials, various liquid fuels (gasoline or liquefied petroleum gas (LPG), aviation fuel, etc.) are hydrocarbons having a carbon number of 2 or higher, and are mainly produced from petroleum resources. However, due to the petroleum resources mainly being the raw materials in the production process of these hydrocarbons, there is concern over geopolitical risks. Furthermore, in a production process or consumption process of these hydrocarbons, due to discharging an abundance of carbon dioxide, it is considered to be a problem also from the viewpoint of global warming. Therefore, the development of a clean process establishing resources for which the regions of the reserves are not limited as raw materials, and having lower carbon dioxide emissions has been sought.

As an example, compared to crude oil and gasoline, liquefied petroleum gas has few emissions of carbon dioxide, which is one cause of global warming, and thus research into suppressing global warming is actively being conducted. For example, Patent Document 1 discloses a production method of liquefied petroleum gas including: a synthesis gas production step of producing synthesis gas with a predetermined composition from a carbon-containing raw material, at least one selected from water, oxygen and carbon dioxide, and a low boiling component; a lower paraffin production step of producing a lower paraffin-containing gas containing a low-boiling component from the synthesis gas; a separation step of separating the low boiling component from the lower paraffin-containing gas; and a recycling step of recycling the low-boiling component to the synthesis gas production step.

However, Patent Document 1 has an object of producing a liquefied petroleum gas having high concentrations of propane and butane from a carbon-containing raw material such as natural gas. For this reason, there has been room for improvement in respect to the global warming prevention.

### Citation List

### Patent Document

Patent Document 1: Japanese Unexamined Patent Application, Publication No. 2006-143752

### DISCLOSURE OF THE INVENTION

### Problems to be Solved by the Invention

An object of the present disclosure is to provide a useful hydrocarbon production method and a useful hydrocarbon production device which can stably produce over a long period of time using carbon dioxide as the main raw material, while achieving a reduction in carbon dioxide and a suppression of emission of carbon dioxide.

### Means for Solving the Problems

[1] A useful hydrocarbon production method includes: a methanation step of generating a first gas containing methane from a mixed gas containing hydrogen and carbon dioxide; a dry reforming step of generating a second gas containing carbon monoxide and hydrogen from the first gas; a useful hydrocarbon generating step of generating a third gas containing a useful hydrocarbon from the carbon monoxide and hydrogen in the second gas; and a recycling step of separating a dry reforming recycle gas containing at least carbon dioxide from the third gas, and supplying the dry reforming recycle gas to the dry reforming step.
[2] In the useful hydrocarbon production method as described in the above [1], the recycling step adjusts a supplied amount of the dry reforming recycle gas to be supplied to the dry reforming step, according to a ratio (M_{HC}/M_{CO2}) of a total carbon mole number M_{HC} of hydrocarbons supplied to the dry reforming step relative to a total carbon mole number M_{CO2} of carbon dioxide in the first gas and carbon dioxide in the dry reforming recycle gas supplied to the dry reforming step.
[3] In the useful hydrocarbon production method as described in the above [1] or [2], a CO₂ concentration of the dry reforming recycle gas is 40% or more.
[4] The useful hydrocarbon production method as described in any one of the above [1] to [3] further includes a hydrogen supplying step of supplying hydrogen to the useful hydrocarbon generating step.
[5] The useful hydrocarbon production method as described in any one of the above [1] to [4] further includes a hydrogen recycling step of separating hydrogen from the third gas and supplying the hydrogen to the useful hydrocarbon generating step.
[6] In the useful hydrocarbon production method as described in the above [5], the hydrogen recycling step adjusts a supplied amount of hydrogen to be supplied to the useful hydrocarbon generating step according to a mole number M_{H} of hydrogen in the useful hydrocarbon generating step.
[7] The useful hydrocarbon production method as described in any one of the above [1] to [6] further includes a moisture removing step of removing moisture contained in the first gas discharged from the methanation step.
[8] The useful hydrocarbon production method as described in any one of the above [1] to [7] further includes an adjusting step of adjusting a mole ratio (M_{H}/M_{CO}) of a mole number M_{H} of hydrogen relative to a mole number M_{CO} of carbon monoxide in the second gas obtained in the dry reforming step.
[9] The useful hydrocarbon production method as described in any one of the above [1] to [8] further includes a combusting step of separating a substance containing carbon atoms from the third gas and combusting the substance containing carbon atoms to generate carbon dioxide, in which the recycling step supplies the dry reforming recycle gas and carbon dioxide generated in the combusting step to the dry reforming step.
[10] In the useful hydrocarbon production method as described in any one of the above [1] to [9], the useful hydrocarbon is at least one type of hydrocarbon selected from the group consisting of olefins, aromatic hydrocarbons, gasoline, liquefied petroleum gas and liquid hydrocarbons.
[11] In the useful hydrocarbon production method as described in any one of the above [1] to [9], the useful hydrocarbon is liquefied petroleum gas.
[12] In the useful hydrocarbon production method as described in any one of the above [1] to [9], the useful hydrocarbon is liquefied petroleum gas, and hydrocarbons other than C3 to C4 hydrocarbons are included in the dry reforming recycle gas.
[13] In the useful hydrocarbon production method as described in any one of the above [1] to [9], the useful hydrocarbon is an olefin, and hydrocarbons other than a target olefin are included in the dry reforming recycle gas.
[14] In the useful hydrocarbon production method as described in the above [13], carbon monoxide is further included in the dry reforming recycle gas.
[15] In the useful hydrocarbon production method as described in any one of the above [1] to [9], the useful hydrocarbon is an aromatic hydrocarbon, and hydrocarbons other than a target aromatic hydrocarbon are included in the dry reforming recycle gas.
[16] In the useful hydrocarbon production method as described in the above [15], carbon monoxide is further included in the dry reforming recycle gas.
[17] In the useful hydrocarbon production method as described in any one of the above [1] to [9], the useful hydrocarbon is gasoline, and hydrocarbons other than gasoline are included in the dry reforming recycle gas.
[18] In the useful hydrocarbon production method as described in the above [17], carbon monoxide is further included in the dry reforming recycle gas.
[19] In the useful hydrocarbon production method as described in any one of the above [1] to [9], the useful hydrocarbon is a liquid hydrocarbon, and C4 or lower hydrocarbons are included in the dry reforming recycle gas.
[20] In the useful hydrocarbon production method as described in the above [19], carbon monoxide is further included in the dry reforming recycle gas.
[21] A useful hydrocarbon production device includes: a methanation unit that generates a first gas containing methane from a mixed gas containing hydrogen and carbon dioxide; a dry reforming unit that generates a second gas containing carbon monoxide and hydrogen from the first gas; a useful hydrocarbon generating unit that generates a third gas containing a useful hydrocarbon from the carbon monoxide and hydrogen in the second gas; and a recycling unit that separates a dry reforming recycle gas containing at least carbon dioxide from the third gas and supplies the dry reforming recycle gas to the dry reforming unit.

### Effects of the Invention

According to the present disclosure, it is possible to provide a useful hydrocarbon production method and a useful hydrocarbon production device which can stably produce over a long period of time using carbon dioxide as the main raw material, while achieving a reduction in carbon dioxide and a suppression of emission of carbon dioxide.

### BRIEF DESCRIPTION OF THE DRAWINGS

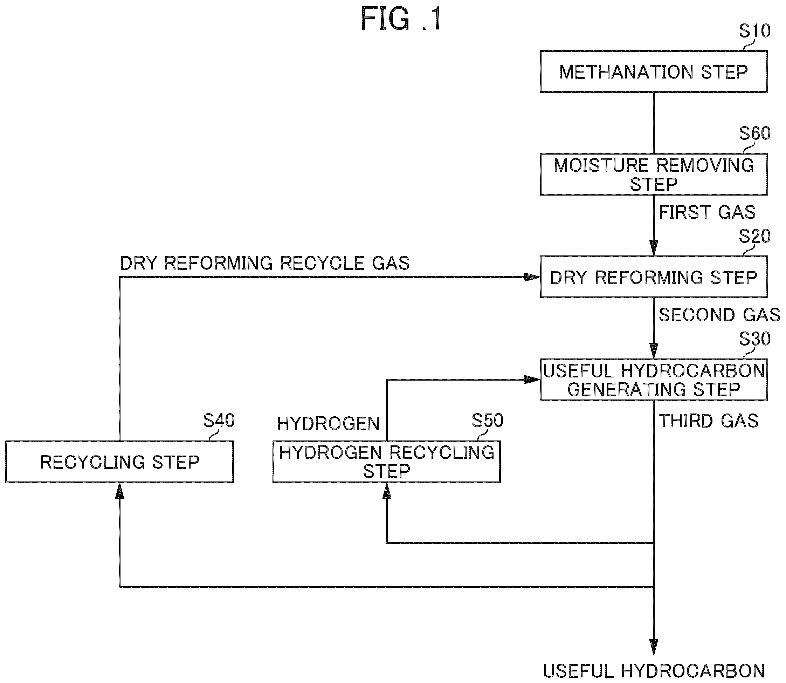

FIG. 1 is a block diagram showing an example of a useful hydrocarbon production method according to an embodiment.

### PREFERRED MODE FOR CARRYING OUT THE INVENTION

Hereinafter, an embodiment of the present invention will be described in detail while referencing the drawing.

As a result of intensive investigation, the present inventors have found that, by using carbon dioxide as one of the main raw materials, and further recycling within the system carbon dioxide, which is one of the principal byproducts, it is possible to stably produce useful hydrocarbons over a long period of time, while achieving a reduction in carbon dioxide and a suppression of emission of carbon dioxide, and based on this knowledge, arrived at completion of the present disclosure.

A useful hydrocarbon production method of an embodiment includes: a methanation step of generating a first gas containing methane from a mixed gas containing hydrogen and carbon dioxide; a dry reforming step of generating a second gas containing carbon monoxide and hydrogen from the first gas; a useful hydrocarbon generating step of generating a third gas containing a useful hydrocarbon from the carbon monoxide and hydrogen in the second gas; and a recycling step of separating a dry reforming recycle gas containing at least carbon dioxide from the third gas and supplying the dry reforming recycle gas to the dry reforming step.

A useful hydrocarbon production device of an embodiment includes: a methanation unit that generates a first gas containing methane from a mixed gas containing hydrogen and carbon dioxide; a dry reforming unit that generates a second gas containing carbon monoxide and hydrogen from the first gas; a useful hydrocarbon generating unit that generates a third gas containing a useful hydrocarbon from the carbon monoxide and hydrogen in the second gas; and a recycling unit that separates a dry reforming recycle gas containing at least carbon dioxide from the third gas and supplies the dry reforming recycle gas to the dry reforming unit.

### (Useful Hydrocarbon Production Method)

First, a useful hydrocarbon production method according to an embodiment will be described. FIG. 1 is a block diagram showing an example of a useful hydrocarbon production method according to the embodiment.

As shown in FIG. 1, the useful hydrocarbon production method includes a methanation step S10, a dry reforming step S20, a useful hydrocarbon generating step S30 and a recycling step S40.

The methanation step S10 generates a first gas containing methane from a mixed gas containing hydrogen and carbon dioxide. For example, using a methanation catalyst, hydrogen and carbon dioxide are made to react and generate a first gas containing methane. The first gas contains methane, hydrogen and carbon dioxide as unreacted substances, as well as water as a byproduct, etc.

So long as being a catalyst which is well known as a methanation catalyst, it can be utilized as the methanation catalyst. For example, as described in Jiajian Gao et al., Recent advances in methanation catalysts for the production of synthetic natural gas, RCS Advances, 5 (2015) 22759, it is possible to contain at least one type selected from Ru, Ni, Co, Fe and Mo, which are metals having activity in methanation, and as the carrier, to use a carrier consisting of at least one type selected from MgO, AL₂O₃, SiO₂, CeO₂, CaO, ZrO₂, TiO₂, La₂O₃, MCM-41 and KIT-6. However, the methanation catalyst is not limited to these.

The heating temperature of the methanation catalyst is appropriately set according to the type of methanation catalyst, supplied amount of mixed gas, content ratio of hydrogen and carbon dioxide contained in the mixed gas, etc. For example, the CH₄ yield in the methanation step is desirably 20% or more. When the CH₄ yield is 20% or more, the CH₄ supplied to the dry reforming step S20 can be sufficiently maintained, and as a result thereof, the useful hydrocarbon can be efficiently generated in the useful hydrocarbon generating step S30. Regarding the heating temperature of the methanation catalyst in order to achieve 20% or more of CH₄ yield, the lower limit value is preferably 180°C or higher, and more preferably 200°C or higher, and the upper limit value is preferably 600°C or lower, and more preferably 500°C or lower.

In the case of the CH₄ yield in the methanation step being less than 20%, since the CH₄ supplied to the dry reforming step S20 cannot be sufficiently secured, it is not preferable.

In the dry reforming step S20 performed after the methanation step S10, the second gas containing carbon monoxide and hydrogen is generated from the first gas generated in the methanation step S10. In the dry reforming step S20, carbon monoxide and hydrogen are synthesized from the methane and carbon dioxide contained in the first gas by dry reforming, for example as in the following Formula (1). In addition, hydrocarbons other than methane may also be included in the dry reforming recycle gas supplied to the dry reforming step S20, and the hydrocarbons serving as the raw material of dry reforming is not limited to methane. Therefore, when generalizing Formula (1) to saturated hydrocarbons, for example, it becomes the following Formula (2). At this time, carbon contained in the hydrocarbons and carbon dioxide as raw materials react in equal amounts.

CH₄+CO₂→2CO+2H₂ ··· Formula (1)

CₙH₂ₙ₊₂+nCO₂→2nCO+ (n+1)H₂ (n≧1) ··· Formula (2)

The dry reforming step S20 uses a catalyst for synthesizing carbon monoxide and hydrogen from the methane and carbon dioxide contained in the first gas by dry reforming (hereinafter also referred to as simply as dry reforming catalyst). In the dry reforming step S20, by heating the dry reforming catalyst while supplying the first gas to the dry reforming catalyst, the supplied methane and carbon dioxide in the first gas react on the dry reforming catalyst, to generate the second gas containing carbon monoxide and hydrogen.

The dry reforming catalyst is not particularly limited; however, from the viewpoint of exhibiting catalytic activity over a long period of time to generate carbon monoxide and hydrogen, for example, the dry reforming catalyst is preferably a catalyst structure including a carrier of a porous structure configured by a zeolite-type compound, and at least one catalytic substance existing within the carrier, in which the carrier has passages communicating with each other, the ratio (L/d ratio) of the long-side dimension L of the carrier relative to the thickness dimension d of the carrier is 5.0 or more, and the catalytic substance exists at least in the passages of the carrier.

Regarding such a catalyst structure, the L/d ratio of the carrier is 5.0 or more, preferably 5.0 or more and 35.0 or less, and more preferably 7.0 or more and 25.0 or less. When the L/d ratio of the carrier is 5.0 or more, it is possible to improve the catalytic activity. In addition, when the L/d ratio is 35.0 or less, it is possible to improve the manufacturing yield of the catalyst structure.

In addition, regarding such a catalyst structure, the average particle size of the catalytic substance is preferably 0.08 nm or more and 50.00 nm or less. When the average particle size of the catalytic substance is within the above-mentioned range, the catalytic activity sufficiently increases, and it is possible to improve the catalytic activity as the average particle size decreases. In addition, from the viewpoint of a balance of high catalytic activity and coking resistance (catalyst deterioration resistance), the average particle size of the catalytic substance is preferably 9.00 nm or less, and more preferably 4.50 nm or less.

In addition, so long as being a catalyst which is well known as a dry reforming catalyst, it can be utilized as the dry reforming catalyst. For example, as described in Yuche Gao et al., A review of recent developments in hydrogen production via biogas dry reforming, Energy Conversion and Management, 171 (2018) 133-155, it is possible to include at least one type selected from Ir, Ru, Rh, Pt, Pd, Ni, Co, and Fe, which are metals having activity in dry reforming, and as the carrier, to use a carrier consisting of at least one type selected from MgO, Al₂O₃, SiO₂, CeO₂, CaO, ZrO₂, TiO₂, La₂O₃, ZnO, silicalite-1, MCM-41 and SBA-15. However, the dry reforming catalyst is not particularly limited to these.

The heating temperature of the dry reforming catalyst is appropriately set according to the type of dry reforming catalyst, supplied amount of the first gas, content ratio of methane and carbon dioxide contained in the first gas, etc. For example, for the heating temperature of the dry reforming catalyst, the lower limit value is preferably 400°C or higher, and more preferably 600°C or higher, and the upper limit value is preferably 1000°C or lower, and more preferably 900°C or lower.

A synthesis gas containing at least carbon monoxide and hydrogen is contained in the second gas generated in the dry reforming step S20. In addition to carbon monoxide and hydrogen (synthesis gas), methane and other hydrocarbons, and carbon dioxide that are unreacted substances, water which is a byproduct, etc., are contained in the second gas.

The content ratio of carbon monoxide and hydrogen contained in the second gas can be appropriately adjusted according to dry reforming conditions such as the heating temperature of the dry reforming catalyst, supplied amount of the first gas, supplied amount of a dry reforming recycle gas described later, content ratio of methane contained in the first gas, and content ratio of hydrocarbon and carbon dioxide contained in the dry reforming recycle gas.

In the useful hydrocarbon generating step S30 performed after the dry reforming step S20, a third gas containing the useful hydrocarbon is generated from the carbon monoxide and hydrogen in the second gas. More specifically, in the useful hydrocarbon generating step S30, by a useful hydrocarbon synthesis reaction represented by Formula (3) below, the second gas containing the useful hydrocarbon is generated from the carbon monoxide and hydrogen in the second gas generated in the dry reforming step S20.

CO+2H₂→ hydrocarbon ... Formula (3)

In this way, the useful hydrocarbon production method can produce the useful hydrocarbon using carbon dioxide as a principal raw material which is a starting material. In addition, as necessary, the useful hydrocarbon may be separated from the third gas.

The useful hydrocarbon is contained in the third gas generated in the useful hydrocarbon generating step S30. In addition, in addition to the useful hydrocarbon, the third gas contains methane, carbon dioxide, carbon monoxide and hydrogen, which are unreacted substances in the methanation step S10, the dry reforming step S20 and the useful hydrocarbon generating step S30, and water and hydrocarbons other than the useful hydrocarbon and methane, etc. that are byproducts of these steps.

In the recycling step S40 performed after the useful hydrocarbon generating step S30, the dry reforming recycle gas containing at least carbon dioxide is separated from the third gas generated in the useful hydrocarbon generating step S30, and the dry reforming recycle gas is supplied to the dry reforming step S20. The dry reforming recycle gas supplied to the dry reforming step S20 is used as a raw material of the dry reforming performed in the dry reforming step S20.

As shown in the above Formulas (1) and (2), in the dry reforming step S20, carbon monoxide and hydrogen are generated from hydrocarbons and carbon dioxide (1:1 by carbon mole ratio) in the same ratios (carbon basis). On the other hand, in the dry reforming step S20, coking of the dry reforming catalyst tends to occur as the proportion of carbon dioxide relative to hydrocarbon decreases, and as a result, the activity of the dry reforming catalyst declines and the generated amount of the useful hydrocarbon decreases. Furthermore, since the carbon dioxide is deficient, the efficiency of the dry reforming declines.

Therefore, in the recycling step S40, by supplying the dry reforming recycle gas containing carbon dioxide, which is an impurity (off gas) in the third gas generated in the useful hydrocarbon generating step S30, to the dry reforming step S20, since it is possible to suppress coking of the dry reforming catalyst caused by a decrease in the proportion of carbon dioxide relative to hydrocarbons, the activity decline in the dry reforming catalyst can be suppressed. Furthermore, the efficiency decline in the dry reforming can be suppressed. For the reasons, the useful hydrocarbon can be stably produced over a long period of time, while achieving a reduction in carbon dioxide and a suppression of emission of carbon dioxide. In addition, due to reusing the dry reforming recycle gas containing the carbon dioxide generated in the useful hydrocarbon generating step S30 as a raw material of the dry reforming step S20, the environmental burden can be reduced.

In addition, the recycling step S40 preferably adjusts the supplied amount of the dry reforming recycle gas supplied to the dry reforming step S20, according to the ratio (M_{HC}/M_{CO2}) of the total carbon mole number M_{HC} of hydrocarbons supplied to the dry reforming step S20 relative to the total carbon mole number M_{CO2} of carbon dioxide in the first gas and carbon dioxide in the dry reforming recycle gas supplied to the dry reforming step S20. Hydrocarbons supplied to the dry reforming step S20 are methane in the first gas and hydrocarbons in the dry reforming recycle gas.

When the ratio (M_{HC}/M_{CO2}) in the dry reforming step S20 is large, there is a possibility of causing the occurrence of coking of the dry reforming catalyst and a carbon dioxide deficiency. For this reason, when the ratio (M_{HC}/M_{CO2}) in the dry reforming step S20 is large, the recycling step S40 can suppress the coking occurrence and the carbon dioxide deficiency in the dry reforming step S20, and thus improve the efficiency of the dry reforming, by increasing the supplied amount of the dry reforming recycle gas supplied to the dry reforming step S20 to decrease the ratio (M_{HC}/M_{CO2}) in the dry reforming step S20. In this way, by adjusting the supplied amount of the dry reforming recycle gas supplied to the dry reforming step S20 in accordance with the ratio (M_{HC}/M_{CO2}) in the dry reforming step S20, it is possible to further improve the efficiency of the dry reforming in the dry reforming step S20.

When adjusting the supplied amount of the dry reforming recycle gas supplied to the dry reforming step S20 to control the ratio (M_{HC}/M_{CO2}) in the dry reforming step S20 preferably to 1.45 or less, and more preferably to 1.05 or less, it is possible to further improve the above effect.

In addition, when adjusting the ratio (M_{HC}/M_{CO2}) of the total carbon mole number M_{HC} of hydrocarbons supplied to the dry reforming step S20 relative to the total carbon mole number M_{CO2} of carbon dioxide in the first gas and carbon dioxide in the dry reforming recycle gas supplied to the dry reforming step S20 preferably to 0.25 or more, and more preferably to 0.40 or more, it is possible to suppress a hydrocarbon deficiency, and thus improve the efficiency of the dry reforming. Furthermore, the emissions of carbon dioxide can be further suppressed. In the case of the above-mentioned ratio (M_{HC}/M_{CO2}) in the dry reforming step S20 being small, the recycling step S40 increases the ratio (M_{HC}/M_{CO2}) by making an increase in the hydrocarbon amount in the dry reforming recycle gas or a decrease in the carbon dioxide amount in the dry reforming recycle gas.

In addition, the CO₂ concentration of the dry reforming recycle gas supplied to the dry reforming step S20 by the recycling step S40 is preferably 40% or more, more preferably 50% or more, and even more preferably 60% or more. When the CO₂ concentration of the dry reforming recycle gas is 40% or more, it is possible to sufficiently suppress the coking occurrence and the carbon dioxide deficiency in the dry reforming step S20.

Furthermore, the hydrocarbons supplied to the dry reforming step S20 (hydrocarbons contained in the first gas and the dry reforming recycle gas) are constituted from C1 to C4 hydrocarbons which are gaseous at room temperature and pressure (hereinafter also referred to lower hydrocarbons), and C5 or higher hydrocarbons (hereinafter also referred to as C5 or higher hydrocarbons). The C5 or higher hydrocarbons are not particularly limited, and are a pure substance or mixture of C5 or higher hydrocarbons excluding the target useful hydrocarbon, and are preferably C5 to C10 hydrocarbons excluding the target useful hydrocarbon, for example. At this time, the proportion of the carbon mole number of the lower hydrocarbons among the hydrocarbons supplied to the dry reforming step S20 (lower hydrocarbons/lower hydrocarbons + C5 or higher hydrocarbons) is preferably 0.60 or more. When a mixed gas containing hydrocarbons and carbon dioxide in such a composition is supplied to the dry reforming step S20, the coking of the dry reforming catalyst is effectively suppressed, the yield of the useful hydrocarbon generated in the useful hydrocarbon generating step S30 improves, and thus it is possible to stably produce the useful hydrocarbon.

In addition, the useful hydrocarbon production method of the embodiment preferably further includes a hydrogen recycling step S50 of separating hydrogen from the third gas generated in the useful hydrocarbon generating step S30 and supplying the hydrogen to the useful hydrocarbon generating step S30.

As shown in the above Formula (3), in the useful hydrocarbon generating step S30, twice as much hydrogen as carbon monoxide is required. On the other hand, the proportions of carbon monoxide to hydrogen in the second gas generated in the dry reforming step S20 are equal or carbon monoxide is more abundant, as shown in the above Formulas (1) and (2). In this way, since the carbon monoxide and hydrogen are generated in the same amounts, or carbon monoxide is generated in a greater amount than hydrogen in the dry reforming step S20, and twice as much hydrogen than carbon monoxide is required in the useful hydrocarbon generating step S30, when performing the useful hydrocarbon generating step S30 over a long period of time, it may lead to a hydrogen deficiency. As a result thereof, since carbon monoxide becomes excessive, the generated amount of the useful hydrocarbon obtained in the useful hydrocarbon generating step S30 declines.

Therefore, in the hydrogen recycling step S50, by supplying to the useful hydrocarbon generating step S30 hydrogen, which is an impurity (off gas) in the third gas generated in the useful hydrocarbon generating step S30, it is possible to compensate for the insufficient amount of hydrogen, which is the raw material for the useful hydrocarbon synthesis reaction conducted in the useful hydrocarbon generating step S30. In this way, by way of the hydrogen recycling step S50, since it is possible to suppress a hydrogen deficiency due to the useful hydrocarbon generating step S30 over a long period of time, the useful hydrocarbon generating step S30 can be stably performed over a long period of time. For this reason, it is possible to efficiently produce the useful hydrocarbon over a long period of time. In addition, since the hydrogen discharged from the useful hydrocarbon generating step S30 is reused as a raw material of the useful hydrocarbon generating step S30, the environmental burden can be reduced.

In addition, the useful hydrocarbon production method of the embodiment may further include a hydrogen supplying step (not shown) of supplying hydrogen to the useful hydrocarbon generating step S30. While the hydrogen recycling step S50 recycles hydrogen within the system, the hydrogen supplying step supplies hydrogen from outside the system to the useful hydrocarbon generating step S30 in the system. For example, the hydrogen generated by electrolysis of water or like is supplied to the useful hydrocarbon generating step S30.

In addition, the hydrogen recycling step S50 preferably adjusts the supplied amount of hydrogen to be supplied to the useful hydrocarbon generating step S30 according to the mole number M_{H} of hydrogen in the useful hydrocarbon generating step S30, and more preferably adjusts the supplied amount of hydrogen to be supplied to the useful hydrocarbon generating step S30 according to the above-mentioned mole number M_{H}, as well as the type and synthesis reaction of the useful hydrocarbon to be produced.

In the case of producing liquefied petroleum gas as the useful hydrocarbon, for example, if the mole number M_{H} of hydrogen in the useful hydrocarbon generating step S30 is large, the useful hydrocarbon synthesis reaction is favorably carried out. On the other hand, if the mole number M_{H} of hydrogen in the useful hydrocarbon generating step S30 is small, there is a possibility of a hydrogen deficiency arising in the useful hydrocarbon synthesis reaction. For this reason, when the mole number M_{H} of hydrogen in the useful hydrocarbon generating step S30 is small, if the hydrogen recycling step S50 increases the supplied amount of hydrogen to be supplied to the useful hydrocarbon generating step S30, it is possible to suppress the hydrogen deficiency in the useful hydrocarbon generating step S30.

In this way, it is possible to more efficiently produce the target useful hydrocarbon by adjusting, in the hydrogen recycling step S50, the supplied amount of hydrogen to be supplied to the useful hydrocarbon generating step S30 according to the mole number M_{H} of hydrogen in the useful hydrocarbon generating step S30, such as increasing the supplied amount of hydrogen to be supplied to the useful hydrocarbon generating step S30 when the mole number M_{H} of hydrogen in the useful hydrocarbon generating step S30 is small.

For the same reason as the hydrogen recycling step S50, also in the hydrogen supplying step, it is preferable to adjust the supplied amount of hydrogen to be supplied to the useful hydrocarbon generating step S30 according to the mole number M_{H} of hydrogen in the useful hydrocarbon generating step S30, and it is more preferable to adjust the supplied amount of hydrogen to be supplied to the useful hydrocarbon generating step S30 according to the above-mentioned mole number M_{H}, as well as the type and synthesis reaction of the useful hydrocarbon to be produced.

In addition, the hydrogen recycling step S50 preferably adjusts the supplied amount of hydrogen to be supplied to the useful hydrocarbon generating step S30 according to the mole ratio (M_{H}/M_{CO}) of the mole number M_{H} of hydrogen relative to the mole number M_{CO} of carbon monoxide in the useful hydrocarbon generating step S30.

Regarding the mole ratio (M_{H}/M_{CO}) in the useful hydrocarbon generating step S30, if the proportion of hydrogen relative to carbon monoxide is small, there is a possibility of a hydrogen deficiency arising. For this reason, when the mole ratio (M_{H}/M_{CO}) in the useful hydrocarbon generating step S30 is small, if the hydrogen recycling step S50 increases the supplied amount of hydrogen to be supplied to the useful hydrocarbon generating step S30, the hydrogen recycling step S50 can suppress the hydrogen deficiency in the useful hydrocarbon generating step S30. In this way, by the hydrogen recycling step S50 adjusting the supplied amount of hydrogen to be supplied to the useful hydrocarbon generating step S30 according to the mole ratio (M_{H}/M_{CO}) in the useful hydrocarbon generating step S30, it is possible to more reliably suppress the hydrogen deficiency in the useful hydrocarbon generating step S30.

For the same reason as the hydrogen recycling step S50, for the hydrogen supplying step, it is preferable to adjust the supplied amount of hydrogen to be supplied to the useful hydrocarbon generating step S30 according to the mole ratio (M_{H}/M_{CO}) of the mole number M_{H} of hydrogen relative to the mole number M_{CO} of carbon monoxide in the useful hydrocarbon generating step S30.

In addition, the useful hydrocarbon production method of the embodiment may further include an adjusting step (not shown) of adjusting the mole ratio (M_{H}/M_{CO}) of the mole number M_{H} of hydrogen relative to the mole number M_{CO} of carbon monoxide in the second gas obtained in the dry reforming step S20. The adjusting step is performed before the dry reforming step S20.

The adjusting step adjusts the mole ratio (M_{H}/M_{CO}) in the second gas obtained in the dry reforming step S20 by supplying steam to the dry reforming step S20. In the adjusting step, the mole ratio (M_{H}/M_{CO}) in the second gas is adjusted according to the conditions of the useful hydrocarbon synthesis reaction conducted in the useful hydrocarbon generating step S30.

If the second gas having the mole ratio (M_{H}/M_{CO}) adjusted to preferably 1.0 or more and 3.0 or less by the adjusting step is supplied to the useful hydrocarbon generating step S30, since it is possible to suppress the hydrogen deficiency due to the useful hydrocarbon generating step S30 over a long period of time, the useful hydrocarbon generating step S30 can be stably performed over a long period of time. For this reason, the target useful hydrocarbon can be efficiently produced over a long period of time.

In addition, the useful hydrocarbon production method of the embodiment may further include a combusting step (not shown) of separating a substance containing carbon atoms from the third gas, and combusting the substance containing carbon atoms to generate carbon dioxide. In this case, the recycling step S40 supplies the dry reforming recycle gas and the carbon dioxide generated in the combusting step to the dry reforming step S20.

For example, in the case of producing liquefied petroleum gas as the useful hydrocarbon, in addition to the liquefied petroleum gas, the third gas discharged from the useful hydrocarbon generating step S30 contains, as impurities, substances containing carbon atoms such as methane, carbon dioxide, carbon monoxide, methanol, dimethyl ether, C1 to C2 hydrocarbons, and C5 or higher hydrocarbons. The combusting step separates the substance containing carbon atoms in the third gas discharged from the useful hydrocarbon generating step S30 from the third gas, and combusts the separated substance containing carbon atoms to generate carbon dioxide.

The substance containing carbon atoms to be combusted in the combusting step include at least one substance selected from methane, carbon monoxide, methanol, dimethyl ether, and hydrocarbons other than the target useful hydrocarbon. It should be noted that carbon dioxide may be included or may not be included in the substance containing carbon atoms to be combusted in the combusting step.

The recycling step S40 supplies the carbon dioxide generated in the combusting step to the dry reforming step S20, in addition to the dry reforming recycle gas. The amount of carbon dioxide supplied to the dry reforming step S20 can be increased by the combusting step. For this reason, since the coking occurrence and the carbon dioxide deficiency in the dry reforming step S20 can be further suppressed, the target useful hydrocarbon can be efficiently produced over a long period of time. In addition, it is possible to recover the heat generated in the combusting step, and utilize the heat in the dry reforming step S20 and/or the useful hydrocarbon generating step S30.

In addition, it is preferable to adjust the combustion rate of the combusting step, according to the proportion of hydrocarbons to carbon dioxide supplied to the dry reforming step S20. The combustion rate of the combusting step is the proportion of combusting hydrocarbons excluding the target useful hydrocarbon component in the third gas to convert to carbon dioxide as shown in Formula (4) below, and can be calculated from the following Formula. For example, in the case of the combustion rate of the combusting step being 100%, the combusting step generates carbon dioxide by combusting all hydrocarbons excluding the target useful hydrocarbon component in the third gas.

2CₙH₂ₙ₊₂+(3n+1)O₂→ (2n+2)H₂O+2nCO₂ ... Formula (4)

Combustion rate (%) = 100 - (total hydrocarbon amount (C- mol) excluding target useful hydrocarbon component combusted in combusting step x 100 / total hydrocarbon amount (C-mol) excluding target useful hydrocarbon component in third gas)

In this way, it is possible to adjust the amount of carbon dioxide generated in the combusting step, by varying the combustion rate of the combusting step. For this reason, since it is possible to adjust the combustion rate of the combusting step to adjust the generated amount of carbon dioxide according to the proportion of hydrocarbons to carbon dioxide supplied to the dry reforming step S20, and possible to adjust the amount of carbon dioxide supplied to the dry reforming step S20 by the recycling step S40, the generated amount of the target useful hydrocarbon can be increased.

For example, in the case of producing liquefied petroleum gas in the useful hydrocarbon generating step S30, if the combustion rate of the combusting step is 3% or more, the carbon dioxide deficiency in the dry reforming step S20 can be sufficiently suppressed. In addition, if the combustion rate of the combusting step is 40% or less, the proportion of hydrocarbons to carbon dioxide supplied to the dry reforming step S20 will be favorable. On the other hand, if the combustion rate of the combusting step exceeds 40%, since carbon dioxide becomes excessive relative to hydrocarbons, the unreacted carbon dioxide amount becomes great, and as a result, the generated amount of the useful hydrocarbon decreases. For this reason, if the combustion rate of the combusting step is 3% or more and 40% or less, the useful hydrocarbon can be efficiently produced over a long period of time.

In addition, in the useful hydrocarbon production method of the embodiment, trace amounts of sulfur and halogens may be mixed as impurities in each gas flowing in the system. Since these become poisonous substances for the methanation catalyst used in the methanation step S10, the dry reforming catalyst used in the dry reforming step S20, and the useful hydrocarbon generation catalyst used in the useful hydrocarbon generating step S30, there is a possibility of hindering the catalytic performance of the above catalysts, and may become the cause of damage to equipment. Therefore, the useful hydrocarbon production method of the embodiment may include a desulfurization step, dehalogenation step or the like as appropriate.

In addition, the useful hydrocarbon production method of the embodiment may further include a moisture removing step S60 of removing moisture contained in the first gas discharged from the methanation step S10. The moisture removing step S60 is performed after the methanation step S10 and before the dry reforming step S20. Water may also be contained in the first gas supplied to the dry reforming step S20. By removing the moisture in the first gas by the moisture removing step S60, it is possible to improve the efficiency of the dry reforming performed in the dry reforming step S20, and further suppress a decline in the catalytic performance of the dry reforming catalyst due to moisture.

In addition, the useful hydrocarbon production method of the embodiment may further include a separation step (not shown) of separating unreacted hydrogen contained in the first gas. The separation step is performed after the methanation step S10 and before the dry reforming step S20. The hydrogen separated by the separation step may be recycled as a raw material of the methanation step S10, or may be supplied as a raw material of the useful hydrocarbon generating step S30. The hydrogen, which is an off gas of the methanation step S10, can be effectively utilized by the separation step.

In addition, the useful hydrocarbon production method of the embodiment may include a dehydrating step (not shown) of dehydrating the second gas generated in the dry reforming step S20. The dehydrating step is performed after the dry reforming step S20 and before the useful hydrocarbon generating step S30. Water may be contained in the second gas supplied to the useful hydrocarbon generating step S30. By dehydrating the second gas in the dehydrating step, it is possible to improve the efficiency of the useful hydrocarbon synthesis reaction performed in the useful hydrocarbon generating step S30, and possible to further suppress a decline in the catalytic performance of the useful hydrocarbon generation catalyst due to moisture.

In addition, the useful hydrocarbon production method of the embodiment may include a compressing step (not shown) of compressing the second gas generated in the dry reforming step S20. The compressing step is performed after the dry reforming step S20 and before the useful hydrocarbon generating step S30. By compressing the second gas in the compressing step, since the compressed second gas is supplied to the useful hydrocarbon generating step S30, the efficiency of the useful hydrocarbon synthesis reaction performed in the useful hydrocarbon generating step S30 can be improved.

In addition, the useful hydrocarbon produced by the useful hydrocarbon production method is preferably at least one type of hydrocarbon selected from the group consisting of olefins, conjugated diene hydrocarbons, aromatic hydrocarbons, gasoline, liquefied petroleum gas and liquid hydrocarbons, and is more preferably liquefied petroleum gas.

As the olefins, ethylene and propylene are preferable. As the conjugated diene hydrocarbons, butadiene is preferable. As the aromatic hydrocarbons, benzene, toluene and xylene are preferable. As gasoline, C5 to C12 hydrocarbons are preferable. As the liquefied petroleum gas, C3 to C4 hydrocarbons are preferable. As the liquid hydrocarbons, naphtha, kerosene, jet fuel, light oil and heavy oil, which are C5 or higher hydrocarbons, are preferable.

The useful hydrocarbon production method can synthesize a hydrocarbon mixture with various target useful hydrocarbons as the main component, from carbon monoxide and hydrogen, by various useful hydrocarbon synthesis reactions, with the appropriate catalyst (useful hydrocarbon generation catalyst) and reaction conditions in the useful hydrocarbon generating step S30, according to the type of useful hydrocarbon to be produced.

For example, in the case of producing olefins as the useful hydrocarbon, olefin synthesis reaction is performed using the useful hydrocarbon generation catalyst (olefin generation catalyst) for synthesizing the olefins from carbon monoxide and hydrogen contained in the second gas in the useful hydrocarbon generating step S30. In the useful hydrocarbon generating step S30, by heating the olefin generation catalyst while supplying the second gas to the olefin generation catalyst, the carbon monoxide and hydrogen in the supplied second gas react on the olefin generation catalyst to generate the second gas containing olefins.

From the viewpoint of increasing the generated amount of olefins, the olefin generation catalyst is preferably a catalyst including a methanol synthesis catalytic substance and a ZSM-5-type zeolite catalytic substance.

For such a catalyst, the ZSM-5-type zeolite catalytic substance preferable contains P (phosphorus). If the ZSM-5-type zeolite catalytic substance includes P, since the acid strength of the ZSM-5-type zeolite catalytic substance can be properly controlled, it is possible to curb the growth of excessive hydrocarbon chains, and increase the generated amount of olefins such as ethylene and propylene.

In addition, for such a catalyst, the ratio (mole number of SiO₂/mole number of Al₂O₃) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of olefins. In addition, if the above ratio is 20 or more, it is possible to suppress catalyst degradation such as coking caused by the acid sites becoming too abundant.

The heating temperature of the olefin generation catalyst is appropriately set according to the type of olefin generation catalyst, supplied amount of the second gas, etc. For example, the heating temperature of the olefin generation catalyst is 240°C or higher and 350°C or lower.

For the pressure of the olefin synthesis reaction, the lower limit value is preferably 2.0 MPa or more, more preferably 3.0 MPa or more, and even more preferably 3.5 MPa or more, and the upper limit value is preferably 6.0 MPa or less, more preferably 5.5 MPa or less, and even more preferably 5.0 MPa or less.

At this time, at least olefins which are useful hydrocarbon are contained in the third gas generated in the useful hydrocarbon generating step S30. The third gas contains, in addition to olefins, methane, carbon dioxide, carbon monoxide and hydrogen that are unreacted substances of the dry reforming step S20 and the useful hydrocarbon generating step S30, and methanol, dimethyl ether, and C10 or lower hydrocarbons, etc. that are byproducts of these steps.

The content ratio of olefins contained in the third gas can be appropriately adjusted according to the conditions of the olefin synthesis reaction such as the heating temperature of the olefin generation catalyst and the supplied amount of the second gas.

In the case of the useful hydrocarbon being olefins, hydrocarbons other than the target olefins are preferably contained in the dry reforming recycle gas, and hydrocarbons other than the target olefins and carbon monoxide are more preferably contained therein. If the dry reforming recycle gas contains the above-mentioned substances, the yield of olefins drastically improves.

For example, in the case of producing aromatic hydrocarbons as the useful hydrocarbon, the aromatic hydrocarbon synthesis reaction is performed using the useful hydrocarbon generation catalyst (aromatic hydrocarbon generation catalyst) for synthesizing the aromatic hydrocarbons from the carbon monoxide and hydrogen contained in the second gas in the useful hydrocarbon generating step S30. In the useful hydrocarbon generating step S30, by heating the aromatic hydrocarbon generation catalyst while supplying the second gas to the aromatic hydrocarbon generation catalyst, the carbon monoxide and hydrogen in the supplied second gas react on the aromatic hydrocarbon generation catalyst to generate the third gas containing the aromatic hydrocarbons.

From the viewpoint of increasing the generated amount of aromatic hydrocarbons, the aromatic hydrocarbon generation catalyst is preferably a catalyst including a methanol synthesis catalytic substance and a ZSM-5-type zeolite catalytic substance.

For such a catalyst, it is preferable to support, as the active metal oxide, at least one among MnO, MnCr₂O₄, MnAl₂O₄, MnZrO₄, ZnO, ZnCr₂O₄ and ZnAl₂O₄ on the ZSM-5-type zeolite catalytic substance, and it is more preferable to support at least one among MnO, MnCr₂O₄, MnAl₂O₄, and MnZrO₄ thereon. If the ZSM-5-type zeolite catalytic substance supports the above-mentioned metal oxide, it is possible to increase the generated amount of aromatic hydrocarbons.

In addition, for such a catalyst, the ratio (mole number of SiO₂/ mole number of Al₂O₃) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of aromatic hydrocarbons. In addition, if the above ratio is 20 or more, it is possible to suppress catalyst degradation such as coking caused by the acid sites becoming too abundant.

The heating temperature of the aromatic hydrocarbon generation catalyst is appropriately set according to the type of aromatic hydrocarbon generation catalyst, supplied amount of the second gas, etc. For example, the heating temperature of the aromatic hydrocarbon generation catalyst is 300°C or higher and 600°C or lower.

The pressure of the aromatic hydrocarbon synthesis reaction is preferably 0.1 MPa or more and 6.0 MPa or less.

At this time, at least the aromatic hydrocarbons which are the useful hydrocarbon are contained in the third gas generated in the useful hydrocarbon generating step S30. In addition to the aromatic hydrocarbons, the third gas contains methane, carbon dioxide, carbon monoxide and hydrogen that are unreacted substances of the dry reforming step S20 and the useful hydrocarbon generating step S30, and methanol, dimethyl ether and hydrocarbons other than the aromatic hydrocarbons that is the useful hydrocarbon, etc. which are byproducts of these steps.

The content ratio of aromatic hydrocarbons contained in the third gas can be appropriately adjusted according to the conditions of the aromatic hydrocarbon synthesis reaction such as the heating temperature of the aromatic hydrocarbon generation catalyst and the supplied amount of the second gas.

In the case of the useful hydrocarbon being aromatic hydrocarbons, hydrocarbons other than the target aromatic hydrocarbons are preferably contained in the dry reforming recycle gas, and hydrocarbons other than the target aromatic hydrocarbons and carbon monoxide are more preferably contained therein. If the dry reforming recycle gas contains the above-mentioned substances, the yield of aromatic hydrocarbons drastically improves.

For example, in the case of producing gasoline as the useful hydrocarbon, the gasoline synthesis reaction is performed using the useful hydrocarbon generation catalyst (gasoline generation catalyst) for synthesizing gasoline from the carbon monoxide and hydrogen contained in the second gas in the useful hydrocarbon generating step S30. In the useful hydrocarbon generating step S30, by heating the gasoline generation catalyst while supplying the second gas to the gasoline generation catalyst, the carbon monoxide and hydrogen in the supplied second gas react on the gasoline generation catalyst to generate the third gas containing gasoline.

From the viewpoint of increasing the generated amount of gasoline, the gasoline generation catalyst is preferably a catalyst including a methanol synthesis catalytic substance and a zeolite catalytic substance.

In addition, for such a catalyst, the ratio (mole number of SiO₂/mole number of Al₂O) of the mole number of SiO₂ relative to the mole number of Al₂O₃ contained in the zeolite catalytic substance is preferably at least 12. In addition, the zeolite catalytic substance has a pore diameter formed preferably by up to 12-membered rings, and more preferably by up to 10-membered rings. By replacing a part of the silicon atoms in the silicates constituting the zeolite skeleton of the zeolite catalytic substance with aluminum atoms, since the aluminum atoms serve as acid sites, the zeolite catalytic substance exhibits a function as a solid acid. If the above such zeolite catalytic substance, it is possible to increase the generated amount of gasoline. Such a zeolite catalytic substance is, for example, ZSM-5, ZSM-11, ZSM-12, ZSM-23, ZSM-35 and ZSM-38.

The heating temperature of the gasoline generation catalyst is appropriately set according to the type of gasoline generation catalyst, the supplied amount of the second gas, etc. For example, the heating temperature of the gasoline generation catalyst is 250°C or higher and 500°C or lower, and preferably 300°C or higher and 450°C or lower.

The pressure of the gasoline synthesis reaction is preferably 25 bar or more and 150 bar or less.

At this time, at least gasoline which is the useful hydrocarbon is contained in the third gas generated in the useful hydrocarbon generating step S30. In addition to the gasoline, the third gas contains methane, carbon dioxide, carbon monoxide and hydrogen that are unreacted substances of the dry reforming step S20 and the useful hydrocarbon generating step S30, and methanol, dimethyl ether and hydrocarbons other than the gasoline, etc. that are byproducts of these steps.

The content ratio of gasoline contained in the third gas can be appropriately adjusted according to the conditions of the gasoline synthesis reaction such as the heating temperature of the gasoline generation catalyst and the supplied amount of the second gas.

In the case of the useful hydrocarbon being gasoline, hydrocarbons other than the target gasoline are preferably contained in the dry reforming recycle gas, and hydrocarbons other than the target gasoline and carbon monoxide are more preferably contained therein. If the dry reforming recycle gas contains the above-mentioned substances, the yield of gasoline drastically improves.

For example, in the case of producing liquefied petroleum gas as the useful hydrocarbon, the liquefied petroleum gas synthesis reaction is performed using the useful hydrocarbon generation catalyst (LPG generation catalyst) for synthesizing liquefied petroleum gas from the carbon monoxide and hydrogen contained in the second gas in the useful hydrocarbon generating step S30. In the useful hydrocarbon generating step S30, by heating the LPG generation catalyst while supplying the second gas to the LGP generation catalyst, the carbon monoxide and hydrogen in the supplied second gas react on the LPG generation catalyst to generate the third gas containing liquefied petroleum gas.

From the viewpoint of increasing the generated amount of liquefied petroleum gas and increasing the content ratio of propane contained in the liquefied petroleum gas, the LPG generation catalyst is preferably a catalyst including a methanol synthesis catalytic substance and a ZSM-5-type zeolite catalytic substance.

For such a catalyst, Pt (platinum) or Pt and Pd (palladium) are preferably supported on the ZSM-5-type zeolite catalytic substance. If the ZSM-5-type zeolite catalytic substance supports Pt, or Pt and Pd, it is possible to increase the generated amount of liquefied petroleum gas and the content ratio of propane. For the state of the Pt and Pd supported on the ZSM-5-type zeolite catalytic substance, Pt of metal simple substance and Pd of metal simple substance may mix, Pt and Pd may alloy, or a metal simple substance of at least one among Pt and Pd and an alloy of Pt and Pd may mix.

In addition, for such a catalyst, the ZSM-5-type zeolite catalytic substance preferably contains P. If the ZSM-5-type zeolite catalytic substance contains P, since the acid strength of the ZSM-5-type zeolite catalytic substance can be properly controlled, it is possible to increase the generated amount of liquefied petroleum gas and the content ratio of propane.

In addition, for such a catalyst, the ratio (mole number of SiO₂/ mole number of Al₂O) of the mole number of SiO₂ relative to the mole number of Al₂O₃ contained in the ZSM-5-type zeolite catalytic substance is preferably 20 or more and 60 or less. By replacing a part of the silicon atoms in the silicates constituting the zeolite skeleton of the ZSM-5-type zeolite catalytic substance with aluminum atoms, since the aluminum atoms serve as acid sites, the zeolite catalytic substance exhibits a function as a solid acid. If the above ratio is 60 or less, since the acid sites of the ZSM-5-type zeolite catalytic substance increase, it is possible to increase the generated amount of liquefied petroleum gas and the content ratio of propane. In addition, if the above ratio is 20 or more, it is possible to suppress catalyst degradation such as coking caused by the acid sites becoming too abundant.

The heating temperature of the LPG generation catalyst is appropriately set according to the type of LPG generation catalyst, the supplied amount of the second gas, etc. For example, the heating temperature of the LPG generation catalyst is 240°C or higher and 350°C or lower.

For the pressure of the liquefied petroleum gas synthesis reaction, the lower limit value is preferably 2.0 MPa or more, more preferably 3.0 MPa or more, and even more preferably 3.5 MPa or more, and the upper limit value is preferably 6.0 MPa or less, more preferably 5.5 MPa or less, and even more preferably 5.0 MPa or less.

At this time, at least the liquefied petroleum gas which is the useful hydrocarbon is contained in the third gas generated in the useful hydrocarbon generating step S20. In addition to the liquefied petroleum gas, the third gas contains methane, carbon dioxide, carbon monoxide and hydrogen that are unreacted substances of the dry reforming step S20 and the useful hydrocarbon generating step S30, and water, methanol, dimethyl ether, C1 to C2 hydrocarbons, C5 or higher hydrocarbons, etc. that are byproducts of these steps.

The content ratio of the liquefied petroleum gas contained in the third gas can be appropriately adjusted according to the conditions of the liquefied petroleum gas synthesis reaction such as the heating temperature of the LPG generation catalyst and the supplied amount of the second gas.

In the case of the useful hydrocarbon being liquefied petroleum gas, hydrocarbons other than C3 to C4 hydrocarbons are preferably contained in the dry reforming recycle gas, C1 to C2 hydrocarbons are more preferably contained therein, and ethane is even more preferably contained therein. If the dry reforming recycle gas contains the above-mentioned substances, the yield of liquefied petroleum gas drastically improves.

For example, in the case of producing liquid hydrocarbons as the useful hydrocarbon, the liquid hydrocarbon synthesis reaction is performed using the useful hydrocarbon generation catalyst (liquid hydrocarbon generation catalyst) for synthesizing the liquid hydrocarbons from the carbon monoxide and hydrogen contained in the second gas in the useful hydrocarbon generating step S30. In the useful hydrocarbon generating step S30, by heating the liquid hydrocarbon generation catalyst while supplying the second gas to the liquid hydrocarbon generation catalyst, the carbon monoxide and hydrogen in the supplied second gas react on the liquid hydrocarbon generation catalyst to generate the third gas containing the liquid hydrocarbons.

From the viewpoint of increasing the generated amount of liquid hydrocarbons, the liquid hydrocarbon generation catalyst is preferably an FT (Fischer Tropsch) synthesis catalyst.

As the FT synthesis catalyst, it is preferable to support at least one type of metal having activity against the FT reaction on the support. The support is preferably Al₂O₃ or SiO₂. In addition, the metal having activity against the FT reaction is preferably Ru, Co, Fe or Ni, and is more preferably Ru or Co. In the case of the above metal being Ru, the FT synthesis catalyst preferably contains Ru in 0.5% by mass or more and 5.0% by mass or less in terms of the metal. In the case of the above metal being Co, the FT synthesis catalyst preferably contains Co in 5.0% by mass or more and 40.0% by mass or less in terms of the metal.

In addition, the FT synthesis catalyst is a catalyst structure exemplified as an ideal example of the above-mentioned dry reforming catalyst, and may be a structure in which Co is included inside.

The heating temperature of the liquid hydrocarbon generation catalyst is appropriately set according to the type of liquid hydrocarbon generation catalyst, the supplied amount of the second gas, etc. For example, the heating temperature of the liquid hydrocarbon generation catalyst is 200°C or higher and 350°C or lower, preferably 210°C or higher and 310°C or lower, and more preferably 220°C or higher and 290°C or lower.

The pressure of the liquid hydrocarbon synthesis reaction is preferably 0.5 MPa or more and 10.0 MPa or less, more preferably 0.7 MPa or more and 7.0 MPa or less, and even more preferably 0.8 MPa or more and 5.0 MPa or less.

At this time, at least the liquid hydrocarbons which are the useful hydrocarbon are contained in the third gas generated in the useful hydrocarbon generating step S30. In addition to the liquid hydrocarbons, the third gas contains methane, carbon dioxide, carbon monoxide and hydrogen that are unreacted substances of the dry reforming step S20 and the useful hydrocarbon generating step S30, and water and C4 or lower hydrocarbons, etc. that are byproducts of these steps.

The content ratio of liquid hydrocarbons contained in the third gas can be appropriately adjusted according to the conditions of the liquid hydrocarbon synthesis reaction such as the heating temperature of the liquid hydrocarbon generation catalyst and the supplied amount of the second gas.

In the case of the useful hydrocarbon being liquid hydrocarbons, C4 or lower hydrocarbons are preferably contained in the dry reforming recycle gas, and C4 or lower hydrocarbons and carbon monoxide are more preferably contained therein. If the dry reforming recycle gas contains the above-mentioned substances, the yield of the liquid hydrocarbons drastically improves.

### (Useful Hydrocarbon Production Device)

Next, a useful hydrocarbon production device according to an embodiment will be described. The useful hydrocarbon production device according to the embodiment is a device which performs the useful hydrocarbon production method of the above embodiment.

The useful hydrocarbon production device includes a methanation unit, a dry reforming unit, a useful hydrocarbon generating unit and a recycling unit.

The methanation unit constituting the useful hydrocarbon production device performs mainly the above-mentioned methanation step S10. The methanation unit generates the first gas containing methane from a mixed gas containing hydrogen and carbon dioxide. For example, the hydrogen and carbon dioxide are made to react using the methanation catalyst to generate the first gas containing methane. The first gas contains methane, hydrogen and carbon dioxide as unreacted substances, water as a byproduct, etc.

The dry reforming unit constituting the useful hydrocarbon production device performs mainly the above-mentioned dry reforming step S20. The first gas generated in the methanation unit is supplied to the dry reforming unit. The dry reforming unit includes a dry reforming catalyst (not shown), and a heater (not shown) for heating the dry reforming catalyst. For example, the heater is a heating furnace. The dry reforming catalyst is heated to a predetermined temperature by the heater.

If the first gas is supplied to the dry reforming unit including the dry reforming catalyst in a heated state, the dry reforming unit causes the methane and carbon dioxide in the first gas to react on the dry reforming catalyst to generate the second gas containing carbon monoxide and hydrogen. In this way, the dry reforming is performed on the first gas in the dry reforming unit.

The useful hydrocarbon generating unit constituting the useful hydrocarbon production device performs mainly the above-mentioned useful hydrocarbon generating step S30. The second gas generated by the dry reforming unit is supplied to the useful hydrocarbon generating unit. The useful hydrocarbon generating unit includes a useful hydrocarbon generation catalyst (not shown), and a heater (not shown) for heating the useful hydrocarbon generation catalyst. For example, the heater is a heating furnace. The useful hydrocarbon generation catalyst is heated to a predetermined temperature by the heater.

If the second gas is supplied to the useful hydrocarbon generating unit including the useful hydrocarbon generation catalyst in a heated state, the useful hydrocarbon generating unit causes the carbon monoxide and hydrogen in the second gas to react on the useful hydrocarbon generation catalyst to generate the third gas containing the target useful hydrocarbon. In this way, the useful hydrocarbon synthesis reaction is performed on the second gas in the useful hydrocarbon generating unit.

The recycling unit constituting the useful hydrocarbon production device performs mainly the above-mentioned recycling step S40. The recycling unit separates the dry reforming recycle gas from the third gas generated in the useful hydrocarbon generating unit, and supplies the dry reforming recycle gas to the dry reforming unit. The dry reforming recycle gas supplied to the dry reforming unit can be used as a raw material of the dry reforming performed by the dry reforming unit.

In this way, by the recycling unit supplying the dry reforming recycle gas containing carbon dioxide which is an impurity (off gas) in the third gas generated by the useful hydrocarbon generating unit to the dry reforming unit, it is possible to suppress the coking occurrence and the carbon dioxide deficiency in the dry reforming unit. For this reason, the useful hydrocarbon can be stably produced over a long period of time, while achieving a reduction in carbon dioxide and a suppression of emission of carbon dioxide. In addition, since the dry reforming recycle gas discharged from the useful hydrocarbon generating unit is reused as a raw material of the dry reforming unit, the environmental burden can be reduced.

In addition, the recycling unit preferably adjusts the supplied amount of the dry reforming recycle gas supplied to the dry reforming unit, according to the ratio (M_{HC}/M_{CO2}) of the total carbon mole number M_{HC} of hydrocarbons supplied to the dry reforming unit relative to the total carbon mole number M_{CO2} of carbon dioxide in the first gas and carbon dioxide in the dry reforming recycle gas supplied to the dry reforming unit. Hydrocarbons supplied to the dry reforming unit are methane in the first gas and hydrocarbons in the dry reforming recycle gas.

When the carbon mole ratio (M_{HC}/M_{CO2}) in the dry reforming unit is large, there is a possibility of causing the coking occurrence and the carbon dioxide deficiency in the dry reforming unit. For this reason, when the carbon mole ratio (M_{HC}/M_{CO2}) in the dry reforming unit is large, if the recycling unit increases the supplied amount of the dry reforming recycle gas to be supplied to the dry reforming unit, and decreases the ratio (M_{HC}/M_{CO2}) in the dry reforming unit, it is possible to suppress the coking occurrence and the carbon dioxide deficiency in the dry reforming unit.

In this way, by the recycling unit adjusting the supplied amount of the dry reforming recycle gas to be supplied to the dry reforming unit according to the carbon mole ratio (M_{HC}/M_{CO2}) in the dry reforming unit, the target useful hydrocarbon can be more efficiently produced. When adjusting the supplied amount of the dry reforming recycle gas to be supplied to the dry reforming unit to control the ratio (M_{HC}/M_{CO2}) in the dry reforming unit preferably to 1.45 or less, and more preferably to 1.05 or less, the above-mentioned effect can be further improved.

In addition, when adjusting the ratio (M_{HC}/M_{CO2}) of the total carbon mole number M_{HC} of hydrocarbons supplied to the dry reforming step S20 relative to the total carbon mole number M_{CO2} of carbon dioxide in the first gas and carbon dioxide in the dry reforming recycle gas supplied to the dry reforming step S20 preferably to 0.25 or more, and more preferably to 0.40 or more, it is possible to suppress a hydrocarbon deficiency, and thus improve the efficiency of the dry reforming. Furthermore, the emissions of carbon dioxide can be further suppressed. In the case of the above ratio (M_{HC}/M_{CO2}) in the dry reforming unit being small, the recycling unit increases the ratio (M_{HC}/M_{CO2}) by making an increase in the hydrocarbon amount in the dry reforming recycle gas or a decrease in the carbon dioxide amount in the dry reforming recycle gas.

In addition, the CO₂ concentration of the dry reforming recycle gas supplied to the dry reforming unit by the recycling unit is preferably 40% or more, more preferably 50% or more, and even more preferably 60% or more. When the CO₂ concentration of the dry reforming recycle gas is 40% or more, it is possible to sufficiently suppress the coking occurrence and the carbon dioxide deficiency in the dry reforming unit.

Furthermore, the hydrocarbons supplied to the dry reforming unit are constituted from C1 to C4 hydrocarbons which are gaseous at room temperature and pressure (lower hydrocarbons), and hydrocarbons having a carbon number of 5 or more (C5 or higher hydrocarbons). The C5 or higher hydrocarbons are not particularly limited, and are a pure substance or mixture of C5 or higher hydrocarbons excluding the target useful hydrocarbon, and are preferably C5 to C10 hydrocarbons excluding the target useful hydrocarbon, for example. At this time, the proportion of the carbon mole number of the lower hydrocarbons among the hydrocarbons supplied to the dry reforming unit (lower hydrocarbons/lower hydrocarbons + C5 or higher hydrocarbons) is preferably 0.60 or more. When a mixed gas containing hydrocarbons and carbon dioxide in such a composition is supplied to the dry reforming unit, the coking of the dry reforming catalyst is effectively suppressed, the yield of the useful hydrocarbon generated in the useful hydrocarbon generating unit improves, and thus it is possible to stably produce the useful hydrocarbon.

In addition, the useful hydrocarbon production device of the embodiment may further include a carbon dioxide supplying unit that supplies carbon dioxide to the dry reforming unit. The carbon dioxide supplying unit performs mainly the above-mentioned carbon dioxide supplying step. While the recycling unit recycles carbon dioxide within the system, the carbon dioxide supplying unit supplies carbon dioxide from outside the system to the dry reforming unit in the system. For example, the carbon dioxide supplying unit recovers carbon dioxide outside the system contained in the exhaust gas such as of a plant and in the atmosphere, and supplies the recovered carbon dioxide to the dry reforming unit in the system.

In addition, the useful hydrocarbon production device of the embodiment preferably further includes a hydrogen recycling unit that separates hydrogen from the third gas generated by the useful hydrocarbon generating unit, and supplies the hydrogen to the useful hydrocarbon generating unit. The hydrogen recycling unit performs mainly the above-mentioned hydrogen recycling step S50. If supplying hydrogen which is an impurity in the third gas discharged from the useful hydrocarbon generating unit to the useful hydrocarbon generating unit by the hydrogen recycling unit, it is possible to suppress the hydrogen deficiency in the useful hydrocarbon generating unit. For this reason, the target useful hydrocarbon can be efficiently produced over a long period of time. In addition, due to reusing the hydrogen discharged from the useful hydrocarbon generating unit as a raw material of the useful hydrocarbon generating unit, the environmental burden can be reduced.

In addition, the useful hydrocarbon production device of the embodiment may further include a hydrogen supplying unit that supplies hydrogen to the useful hydrocarbon generating unit. The hydrogen supplying unit performs mainly the above-mentioned hydrogen supplying step. While the hydrogen recycling unit recycles hydrogen within the system, the hydrogen supplying unit supplies hydrogen from outside the system to the useful hydrocarbon generating unit within the system.

In addition, the hydrogen recycling unit preferably adjusts the supplied amount of hydrogen to be supplied to the useful hydrocarbon generating unit according to the mole number M_{H} of hydrogen in the useful hydrocarbon generating unit, and more preferably adjusts the supplied amount of hydrogen to be supplied to the useful hydrocarbon generating unit according to the above-mentioned mole number M_{H}, as well as the type and synthesis reaction of the useful hydrocarbon to be produced.

For example, in the case of producing liquefied petroleum gas as the useful hydrocarbon, if the mole number M_{H} of hydrogen in the useful hydrocarbon generating unit is small, there is a possibility of causing the hydrogen deficiency in the useful hydrocarbon generating unit. For this reason, when the mole number M_{H} of hydrogen in the useful hydrocarbon generating unit is small, if the hydrogen recycling unit increases the supplied amount of hydrogen to be supplied to the useful hydrocarbon generating unit, it is possible to suppress the hydrogen deficiency in the useful hydrocarbon generating unit.

In this way, it is possible to more efficiently produce the target useful hydrocarbon by adjusting, in the hydrogen recycling unit, the supplied amount of hydrogen to be supplied to the useful hydrocarbon generating unit according to the mole number M_{H} of hydrogen in the useful hydrocarbon generating unit, such as the hydrogen recycling unit increasing the supplied amount of hydrogen to be supplied to the useful hydrocarbon generating unit when the mole number M_{H} of hydrogen in the useful hydrocarbon generating unit is small.

For the same reason as the hydrogen recycling unit, also in the hydrogen supplying unit, it is preferable to adjust the supplied amount of hydrogen to be supplied to the useful hydrocarbon generating unit according to the mole number M_{H} of hydrogen in the useful hydrocarbon generating unit, and it is more preferable to adjust the supplied amount of hydrogen to be supplied to the useful hydrocarbon generating unit according to the above-mentioned mole number M_{H}, as well as the type and synthesis reaction of the useful hydrocarbon to be produced.

In addition, the hydrogen recycling unit preferably adjusts the supplied amount of hydrogen to be supplied to the useful hydrocarbon generating unit according to the mole ratio (M_{H}/M_{CO}) of the mole number M_{H} of hydrogen relative to the mole number M_{CO} of carbon monoxide in the useful hydrocarbon generating unit.

Regarding the mole ratio (M_{H}/M_{CO}) of the useful hydrocarbon generating unit, if the proportion of hydrogen relative to carbon monoxide is small, there is a possibility of leading to a hydrogen deficiency. For this reason, when the mole ratio (M_{H}/M_{CO}) in the useful hydrocarbon generating unit is small, if the hydrogen recycling unit increases the supplied amount of hydrogen to be supplied to the useful hydrocarbon generating unit, it is possible to suppress the hydrogen deficiency in the useful hydrocarbon generating unit. In this way, by the hydrogen recycling unit adjusting the supplied amount of hydrogen to be supplied to the useful hydrocarbon generating unit according to the mole ratio (M_{H}/M_{CO}) in the useful hydrocarbon generating unit, it is possible to more reliably suppress the hydrogen deficiency in the useful hydrocarbon generating unit.

For the same reason as the hydrogen recycling unit, for the hydrogen supplying unit, it is preferable to adjust the supplied amount of hydrogen to be supplied to the useful hydrocarbon generating unit according to the mole ratio (M_{H}/M_{CO}) of the mole number M_{H} of hydrogen relative to the mole number M_{CO} of carbon monoxide in the useful hydrocarbon generating unit.

In addition, the useful hydrocarbon production device of the embodiment may further include an adjusting unit that adjusts the mole ratio (M_{H}/M_{CO}) of the mole number M_{H} of hydrogen relative to the mole number M_{CO} of carbon monoxide in the second gas obtained in the dry reforming unit. The adjusting unit performs mainly the above-mentioned adjusting step. The adjusting unit is provided before the dry reforming unit.

The adjusting unit adjusts the mole ratio (M_{H}/M_{CO}) in the second gas discharged from the dry reforming unit. The adjusting unit is preferably a steam drum. The steam drum supplies steam to the dry reforming unit to adjust the mole ratio (M_{H}/M_{CO}) in the second gas. In the adjusting unit, the mole ratio (M_{H}/M_{CO}) in the second gas is adjusted according to the conditions of the useful hydrocarbon synthesis reaction conducted in the useful hydrocarbon generating unit.

If the second gas having the mole ratio (M_{H}/M_{CO}) adjusted to preferably 1.0 or more and 3.0 or less by the adjusting unit is supplied to the useful hydrocarbon generating unit, it is possible to suppress the hydrogen deficiency due to the useful hydrocarbon generating unit. For this reason, the target useful hydrocarbon can be efficiently produced over a long period of time.

In addition, the useful hydrocarbon production device of the embodiment may further include a combusting unit that separates a substance containing carbon atoms from the third gas, and combusts the substance containing carbon atoms to generate carbon dioxide. The combusting unit performs mainly the above-mentioned combusting step. In this case, the recycling unit supplies the dry reforming recycle gas and the carbon dioxide generated in the combusting unit to the dry reforming unit.

The combusting unit separates the substance containing carbon atoms in the third gas discharged from the useful hydrocarbon generating unit from the third gas, and combusts the separated substance containing carbon atoms to generate carbon dioxide. It should be noted that carbon dioxide may be included or may not be included in the substance containing carbon atoms to be combusted in the combusting unit.

In addition to the dry reforming recycle gas, the recycling unit also supplies the carbon dioxide generated in the combusting unit to the dry reforming unit. The amount of carbon dioxide supplied to the dry reforming unit can be increased by the combusting unit. For this reason, since the coking occurrence and the carbon dioxide deficiency in the dry reforming unit can be further suppressed, the target useful hydrocarbon can be efficiently produced over a long period of time. In addition, it is possible to recover the heat generated in the combusting unit, and utilize the heat in the dry reforming unit and/or the useful hydrocarbon generating unit.

In addition, it is preferable to adjust the combustion rate of the combusting unit according to the proportion of hydrocarbons to carbon dioxide supplied to the dry reforming unit. By changing the combustion rate of the combusting unit, it is possible to adjust the amount of carbon dioxide generated in the combusting unit. For this reason, since it is possible to adjust the combustion rate of the combusting unit to adjust the generated amount of carbon dioxide according to the proportion of hydrocarbons to carbon dioxide supplied to the dry reforming unit, and possible to adjust the amount of carbon dioxide supplied to the dry reforming unit by the recycling unit, the generated amount of the target useful hydrocarbon can be increased.

If the combustion rate of the combusting unit is 3% or more, the carbon dioxide deficiency in the dry reforming unit can be sufficiently suppressed. In addition, if the combustion rate of the combusting unit is 40% or less, the proportion of hydrocarbons to carbon dioxide supplied to the dry reforming unit will be favorable. On the other hand, if the combustion rate of the combusting unit exceeds 40%, since carbon dioxide becomes excessive relative to hydrocarbons, the unreacted carbon dioxide amount becomes great, and as a result, the generated amount of the useful hydrocarbon decreases. For this reason, if the combustion rate of the combusting unit is 3% or more and 40% or less, the target useful hydrocarbon can be efficiently produced over a long period of time.

In addition, trace amounts of sulfur and halogens may be mixed as impurities in the useful hydrocarbon production device of the embodiment. Since these become poisonous substances for the methanation catalyst used in the methanation unit, the dry reforming catalyst used in the dry reforming unit, and the useful hydrocarbon generation catalyst used in the useful hydrocarbon generating unit, there is a possibility of hindering the catalytic performance of the above catalysts, and may become the cause of damage to equipment. Therefore, the useful hydrocarbon production device of the embodiment may include a desulfurization unit, dehalogenation unit or the like as appropriate.

In addition, the useful hydrocarbon production device of the embodiment may further include a moisture removing unit that removes moisture contained in the first gas discharged from the methanation unit. The moisture removing unit performs mainly the above-mentioned moisture removing step S60. The moisture removing unit is provided between the methanation unit and the dry reforming unit. By removing the moisture in the first gas by the moisture removing unit, it is possible to improve the efficiency of dry reforming performed in the dry reforming unit, and further suppress a decline in the catalytic performance of the dry reforming catalyst due to moisture.

In addition, the useful hydrocarbon production device of the embodiment may further include a separating unit that separates unreacted hydrogen contained in the first gas. The separating unit performs mainly the above-mentioned separating step. The separating unit is provided between the methanation unit and the dry reforming unit. The hydrogen separated by the separating unit may be recycled as a raw material of the methanation unit, or may be supplied as a raw material of the useful hydrocarbon generating unit. The hydrogen, which is an off gas of the methanation unit, can be effectively utilized by the separating unit.

In addition, the useful hydrocarbon production device of the embodiment may include a dehydrating unit that dehydrates the second gas generated in the dry reforming unit. The dehydrating unit performs mainly the above-mentioned dehydrating step. The dehydrating unit is provided between the dry reforming unit and the useful hydrocarbon generating unit. By the dehydrating unit dehydrating the second gas, it is possible to improve the efficiency of the useful hydrocarbon synthesis reaction performed in the useful hydrocarbon generating unit, and possible to further suppress a decline in the catalytic performance of the useful hydrocarbon generation catalyst due to moisture.

In addition, the useful hydrocarbon production device of the embodiment may include a compressing unit that compresses the second gas generated in the dry reforming unit. The compressing unit performs mainly the above-mentioned compressing step. The compressing unit is provided between the dry reforming unit and the useful hydrocarbon generating unit. By the compressing unit compressing the second gas, since the compressed second gas is supplied to the useful hydrocarbon generating unit, it is possible to improve the efficiency of the synthesis reaction of the target useful hydrocarbon performed in the useful hydrocarbon generating unit.

In addition, the useful hydrocarbon produced by the useful hydrocarbon production device is similar to the useful hydrocarbon produced by the above-mentioned useful hydrocarbon production method, and is preferably at least one type of hydrocarbon selected from the group consisting of olefins, aromatic hydrocarbons, gasoline, liquefied petroleum gas and liquid hydrocarbons, and is more preferably liquefied petroleum gas.

The useful hydrocarbon production device can synthesize a hydrocarbon mixture with various target useful hydrocarbons as the main component, from carbon monoxide and hydrogen, by various useful hydrocarbon synthesis reactions, with the appropriate catalysts (useful hydrocarbon generation catalyst) and reaction conditions in the useful hydrocarbon generating unit, according to the type of useful hydrocarbon to be produced. The various useful hydrocarbon synthesis reactions performed in the useful hydrocarbon generating unit have similar conditions as the useful hydrocarbon generating step S30 of the above-described useful hydrocarbon production method.

According to the above described embodiment, by using carbon dioxide as the main component of the starting material, and further recycling carbon dioxide which is one of the principal byproducts within the system, it is possible to stably produce a useful hydrocarbon over a long period of time, while achieving a reduction in carbon dioxide and a suppression of emission of carbon dioxide.

Although embodiments have been explained above, the present invention is not to be limited to the above embodiments, and encompasses every embodiment included in the gist of the present disclosure and scope of the claims, and various modifications thereto are possible within the scope of the present disclosure.

### EXAMPLES

Next, examples and comparative examples will be described; however, the present invention is not to be limited to these examples.

### (Examples 1-1 to 1-6)

Liquefied petroleum gas, which is a useful hydrocarbon, was produced by the useful hydrocarbon production method including the methanation step, the dry reforming step, the useful hydrocarbon generating step, the recycling step and the hydrogen recycling step. More specifically, it was produced as follows.

A fixed bed flow reactor was used in the methanation step. In addition, a quartz glass reaction tube was used. Using a methanation catalyst (Ni/Al₂O₃), the flowrate of mixed gas containing hydrogen and carbon dioxide (mixed gas with hydrogen : carbon dioxide by mole ratio = 4:1) was set to 12.5 ml/min at the reaction temperature of 380°C. The methanation catalyst was filled so as to be GHSV = 7000 (1/h), and the methanation catalyst was fixed by packing quartz wool above and below this. The above-mentioned methanation catalyst containing 66% by mass of Ni was used as the methanation catalyst, and a reduction treatment was conducted as a pretreatment on the methanation catalyst for 1.5 hours at 700°C under a hydrogen flow. The first gas was generated in this way. The first gas was analyzed by GC-TCD to measure the generated methane, unreacted hydrogen and carbon dioxide. As a result, the CO₂ conversion rate was 78%.

In the dry reforming step, a fixed bed flow reactor was used. In addition, a quartz glass reaction tube was used. Regarding the feedstock gas consisting of the first gas supplied from the methanation step to the dry reforming step and the dry reforming recycle gas supplied from the recycling step to the dry reforming step, the flowrate was set to 10 ml/min with the composition, the ratio (lower hydrocarbons/(lower hydrocarbons + C5 or higher hydrocarbons)) of lower hydrocarbons relative to the total of lower hydrocarbons and C5 or higher hydrocarbons, and the ratio (M_{HC}/M_{CO2}) shown in Table 1. In addition, regarding the dry reforming recycle gas supplied from the recycling step to the dry reforming step, the composition and the CO₂ concentration shown in Table 1 were set. The dry reforming catalyst was filled so as to be GHSV = 2170 (1/h), and the dry reforming catalyst was fixed by packing quartz wool above and below this. The above-mentioned dry reforming catalyst containing 1% by mass of Ni was used as the dry reforming catalyst, and a reduction treatment was conducted as a pretreatment on the dry reforming catalyst for 1.5 hours at 700°C under a hydrogen flow. In the dry reforming step, the dry reforming was performed at the reaction temperature shown in Table 1 to generate the second gas containing carbon monoxide and hydrogen.

In the useful hydrocarbon generating step, a fixed bed flow reactor was used. In addition, a stainless steel reaction tube was used. The total flowrate of the second gas supplied from the dry reforming step to the useful hydrocarbon generating step and hydrogen supplied from the hydrogen recycling step was set to 37.2 ml/min. At this time, the flowrate of hydrogen to be supplied from the hydrogen recycling step was adjusted so that the ratio (M_{H}/M_{CO}) of hydrogen to carbon monoxide supplied to the useful hydrocarbon generating step became 2.0. The useful hydrocarbon generation catalyst (LPG generation catalyst) was filled so as to be GHSV = 2000 (1/h), and the LPG generation catalyst was fixed by packing quartz wool above and below this. A catalyst prepared by physically mixing, in a 1:1 weight ratio, a methanol synthesis catalytic substance and ZSM-5-type zeolite catalytic substance on which Pt and Pd are supported was used as the LPG generation catalyst, and a reduction treatment was conducted as a pretreatment on the LPG generation catalyst for 2 hours at 380°C under a hydrogen flow. The heating temperature of the LPG generation catalyst was set to 320°C. The pressure in the useful hydrocarbon generating step was set to 5.0 MPa. The third gas containing liquefied petroleum gas was generated in this way.

In the recycling step, the dry reforming recycle gas having the composition and the CO₂ concentration shown in Table 1 was seperated from the third gas and supplied to the dry reforming step.

The useful hydrocarbon production method was started, and immediately after starting (initial stage) and after 7 days, the second gas generated in the dry reforming step was recovered by a gas pack and analyzed by GC-TCD, and the synthesis gas (hydrogen and carbon monoxide) generated in the dry reforming step and the generated amount of carbon dioxide were measured. When the synthesis gas thereby generated immediately after starting (initial stage) and the synthesis gas generated after 7 days were respectively supplied to the useful hydrocarbon generating step, the generated amounts of liquefied petroleum gas and carbon dioxide contained in the third gas were measured. In addition, the activity decline rate of the dry reforming catalyst was calculated by the below Formula, from the change in the generated amount of carbon dioxide in the dry reforming step immediately after start (initial stage) and after 7 days. The activity decline rate represents the percentage of the carbon dioxide conversion rate which has deteriorated from immediately after start to after 7 days, relative to the initial carbon dioxide conversion rate. The results of the activity decline rate are shown in Table 1.Activity decline rate (%) = (initial carbon dioxide conversion rate (%) - post-7-day carbon dioxide conversion rate) × 100/initial carbon dioxide conversion rate (%)

Furthermore, the amount of carbon dioxide contained in the useful hydrocarbon, and the total of the discharge amount of carbon dioxide discharged to outside the system of the process after the dry reforming step were calculated.

### (Comparative Example 1-1)

The liquefied petroleum gas was produced similarly to the above example except for including the methanation step, the dry reforming step and the useful hydrocarbon generating step, but not including the recycling step and the hydrogen recycling step.

The ranking of the LPG generated amount in Table 2 was defined as "excellent" for the LPG generated amount at the initial stage and after 7 days exceeding 2.45 (10⁻¹ mol/mol - raw material CO₂), defined as "great" for greater than 1.70 (10⁻¹ mol/mol - raw material CO₂) and 2.45 (10⁻¹ mol/mol - raw material CO₂) or less, defined as "good" for greater than 1.10 (10⁻¹ mol/mol - raw material CO₂) and 1.70 (10⁻¹ mol/mol - raw material CO₂) or less, and defined as "poor" for 1.10 (10⁻¹ mol/mol - raw material CO₂) or less.

In addition, the ranking of the carbon dioxide discharge amount in Table 2 was defined as "great" for the carbon dioxide discharge amount discharged to outside the process system at the initial stage and after 7 days of 1.40 (10⁻¹ mol/mol - raw material CO₂) or less, defined as "good" for greater than 1.40 (10⁻¹ mol/mol - raw material CO₂) and 2.30 (10⁻¹ mol/mol - raw material CO₂) or less, and defined as "poor" for greater than 2.30 (10⁻¹ mol/mol - raw material CO₂).

In addition, regarding the comprehensive evaluation based on these rankings, among the four items of the LPG generated amount and the carbon dioxide discharge amount at the initial stage and after 7 days, the case of one or more being the rank of excellent and the others being great was given "☆" (star), the case of all four being the rank of great was given "⊙" (bullseye), the case of 1 to 3 being the rank of great and the other(s) being good was given "o" (circle), the case of all four being the rank of good was given "Δ" (triangle), and the case of one or more being the rank of poor was given "x" (cross). These results are shown in Table 2.

**[Table 1]**

| | Dry reforming recycle gas | | | | | | Feedstock gas of dry reforming step | | | | | | | Dry reforming | |
|---|---|---|---|---|---|---|---|---|---|---|---|---|---|---|---|
| | Composition (mole ratio) | | | | | CO₂ concentr ation (%) | Composition (mole ratio) | | | | | Lower hydrocarbons / (lower hydrocarbons + C5 or higher hydrocarbons) (carbon mole ratio) | Ratio (M _{H C} /M_{C O 2}) (carbon mole ratio) | Reaction temperatu re (°C) | Activity decline rate (%) |
| | CO₂ | CH₄ | C₂H₆ | C5 or higher hydrocar bons | CO | | CO₂ | CH₄ | C₂H₆ | C5 or higher hydrocar bons | CO | | | | |
| Example 1-1 | 17.5 | 0.0 | 1.0 | 0.0 | 3.0 | 82 | 6.6 | 6.0 | 0.3 | 0.0 | 0.9 | 1.00 | 0.93 | 800 | 0 |
| Example 1-2 | 20.2 | 1.0 | 3.8 | 0.0 | 3.4 | 71 | 8.0 | 6.0 | 1.2 | 0.0 | 1.1 | 1.00 | 1.05 | 800 | 2 |
| Example 1-3 | 18.8 | 1.0 | 3.8 | 0.0 | 3.4 | 70 | 6.8 | 6.0 | 1.0 | 0.0 | 0.9 | 1.00 | 1.19 | 800 | 8 |
| Example 1-4 | 15.9 | 1.0 | 3.8 | 0.0 | 3.4 | 66 | 5.3 | 6.0 | 0.9 | 0.0 | 0.8 | 1.00 | 1.45 | 800 | 10 |
| Example 1-5 | 35.4 | 0.0 | 1.9 | 1.0 | 6.0 | 80 | 46.0 | 37.7 | 1.9 | 1.0 | 6.0 | 0.88 | 1.02 | 800 | 3 |
| Example 1-6 | 35.4 | 1.8 | 0.5 | 1.0 | 6.0 | 79 | 45.9 | 39.0 | 0.5 | 1.0 | 6.0 | 0.88 | 0.99 | 800 | 11 |
| Example 2-1 | 20.8 | 1.0 | 3.8 | 0.0 | 3.4 | 72 | 8.4 | 6.0 | 1.2 | 0.0 | 1.1 | 1.00 | 1.01 | 800 | 0 |
| Example 2-2 | 22.0 | 1.0 | 3.8 | 0.0 | 3.4 | 73 | 9.2 | 6.0 | 1.3 | 0.0 | 1.2 | 1.00 | 0.93 | 800 | 0 |
| Example 2-3 | 24.4 | 1.0 | 3.3 | 0.0 | 3.4 | 76 | 10.4 | 6.0 | 1.2 | 0.0 | 1.2 | 1.00 | 0.81 | 800 | 0 |
| Comparative Example 1-1 | no recycling | | | | | | 1.0 | 3.5 | 0.0 | 0.0 | 0.0 | 1.00 | 3.55 | 800 | 30 |

**[Table 2]**

| | LPG generation amount | | CO₂ discharge amount | | Combus tion rate (%) | Comprehe nsive evaluation |
|---|---|---|---|---|---|---|
| | Initial stage | After 7 days | Initial stage | After 7 days | | |
| Example 1-1 | Great | Great | Great | Great | 0 | ⊚ |
| Example 1-2 | Great | Great | Great | Great | 0 | ⊚ |
| Example 1-3 | Great | Great | Great | Good | 0 | ○ |
| Example 1-4 | Good | Good | Good | Good | 0 | Δ |
| Example 1-5 | Great | Great | Great | Great | 0 | ⊚ |
| Example 1-6 | Great | Good | Great | Great | 0 | ○ |
| Example 2-1 | Great | Great | Great | Great | 5 | ⊚ |
| Example 2-2 | Excellent | Excellent | Great | Great | 15 | ☆ |
| Example 2-3 | Excellent | Excellent | Great | Great | 35 | ☆ |
| Comparative Example 1-1 | Poor | Poor | Good | Poor | 0 | × |

### (Examples 2-1 to 2-3)

Liquefied petroleum gas was produced similarly to Examples 1-1 to 1-6 except for providing the combusting step after the useful hydrocarbon generating step and before the recycling step, and combusting the hydrocarbons other than the useful hydrocarbon in the third gas at the combustion rate shown in Table 2 to convert into carbon dioxide and supplying carbon dioxide to the recycling step, and changing the respective values of the dry reforming recycle gas and the feedstock gas in accordance with Table 1. The activity decline rate, the generated amount of liquefied petroleum gas, the carbon dioxide discharge amount and the comprehensive evaluation are shown in Tables 1 and 2.

### (Examples 3-1 to 3-3)

Similarly to Examples 1-1 to 1-6, liquefied petroleum gas as the useful hydrocarbon was produced by the useful hydrocarbon production method including the methanation step, the dry reforming step, the useful hydrocarbon generating step, the recycling step and the hydrogen recycling step. However, as shown in Table 3, the CH₄ yield was adjusted by varying the reaction temperature between 320 and 380°C in the methanation step. In addition, the dry reforming recycle gas, the feedstock gas of the dry reforming step and the reaction temperature of the dry reforming were set as shown in Table 3.

### (Comparative Example 3-1)

The liquefied petroleum gas was produced similarly to the above Examples 3-1 to 3-3 except for including the methanation step, the dry reforming step and the useful hydrocarbon generating step, but not including the recycling step and the hydrogen recycling step.

The ranking of the LPG generated amount in Table 4 was defined as "excellent" for the LPG generated amount at the initial stage and after 7 days exceeding 2.45 (10⁻¹ mol/mol - raw material CO₂), defined as "great" for greater than 1.70 (10⁻¹ mol/mol - raw material CO₂) and 2.45 (10⁻¹ mol/mol - raw material CO₂) or less, defined as "good" for greater than 1.10 (10⁻¹ mol/mol - raw material CO₂) and 1.70 (10⁻¹ mol/mol - raw material CO₂) or less, and defined as "poor" for 1.10 (10⁻¹ mol/mol - raw material CO₂) or less.

In addition, the ranking of the carbon dioxide discharge amount in Table 4 was defined as "great" for the carbon dioxide discharge amount discharged to outside the process system at the initial stage and after 7 days of 5.00 (10⁻¹ mol/mol - raw material CO₂) or less, defined as "good" for greater than 5.00 (10⁻¹ mol/mol - raw material CO₂) and 6.00 (10⁻¹ mol/mol - raw material CO₂) or less, and defined as "poor" for greater than 6.00 (10⁻¹ mol/mol - raw material CO₂).

In addition, regarding the comprehensive evaluation based on these rankings, among the four items of the LPG generated amount and the carbon dioxide discharge amount at the initial stage and after 7 days, the case of one or more being the rank of excellent and the others being great was given "☆" (star), the case of all four being the rank of great was given "⊙" (bullseye), the case of 1 to 3 being the rank of great and the other(s) being good was given "o" (circle), the case of all four being the rank of good was given "Δ" (triangle), and the case of one or more being the rank of poor was given "x" (cross). These results are shown in Table 4.

**[Table 3]**

| | Methanation reaction condition and reaction results | | | Dry reforming recycle gas | | | | | | Feedstock gas of dry reforming step | | | | | | | Dry reforming | |
|---|---|---|---|---|---|---|---|---|---|---|---|---|---|---|---|---|---|---|
| | | | | Composition (mole ratio) | | | | | CO₂ concentr ation (%) | Lower hydrocarbons / (lower hydrocarbons + C5 or higher hydrocarbons) (carbon mole ratio) | Composition (mole ratio) | | | | | Ratio (M_{HC} / M_{CO2}) (carbon mole ratio) | Reaction temperat ure (°C) | Activity decline rate (%) |
| | Reaction temperat ure (°C) | CO₂ yield (C-mol%) | CH₄ yield (C-mol%) | CO₂ | CH₄ | C₂H₆ | C5 or higher hydrocar bons | CO | | | CO₂ | CH₄ | C₂H₆ | C5 or higher hydrocar bons | CO | | | |
| Example 3-1 | 340 | 50 | 50 | 39.3 | 1.9 | 7.3 | 1.0 | 6.7 | 70 | 0.87 | 60.2 | 23.1 | 7.3 | 1.0 | 6.7 | 0.72 | 900 | 0.0 |
| Example 3-2 | 380 | 22 | 78 | | | | | | | | 47.1 | 29.4 | 7.3 | 1.0 | 6.7 | 1.05 | 900 | 0.0 |
| Example 3-3 | 320 | 78 | 22 | | | | | | | | 91.4 | 16.8 | 7.3 | 1.0 | 6.7 | 0.40 | 900 | 0.0 |
| Comparative Example 3-1 | 280 | 96 | 4 | no recycling | | | | | | - | 21.5 | 1.0 | 0.0 | 0.0 | 0.0 | 0.05 | 900 | 0.0 |

**[Table 4]**

| | LPG generation amount | | CO₂ discharge amount | | Comprehe nsive evaluation |
|---|---|---|---|---|---|
| | Initial stage | After 7 days | Initial stage | After 7 days | |
| Example 3-1 | Great | Great | Great | Great | ○ |
| Example 3-2 | Excellent | Excellent | Great | Great | ⊚ |
| Example 3-3 | Good | Good | Good | Good | Δ |
| Comparative Example 3-1 | Poor | Poor | Poor | Poor | × |

In Examples 4-1 to 4-4, propylene which is an olefin, aromatic hydrocarbons, gasoline and liquid hydrocarbon were produced in the useful hydrocarbon generating step, by the production method including the methanation step, the dry reforming step, the useful hydrocarbon generating step, the recycling step, the hydrogen recycling step and the carbon dioxide supplying step. The useful hydrocarbon generating step in each example was executed as follows, and carbon dioxide was supplied so as to make the hydrocarbon/carbon dioxide ratio (M_{HC}/M_{CO2}) shown in Table 5 by the carbon dioxide supplying step. Otherwise, the methanation step, the dry reforming step, the recycling step and the hydrogen recycling step were conducted similarly to Examples 1-1 to 1-6 at the conditions shown in Table 5.

### (Example 4-1)

Propylene, which is an olefin, was produced similarly to the above Examples 1-1 to 1-6, except for using a catalyst made by physically mixing, in a 1:1 weight ratio, a methanol synthesis catalytic substance and a ZSM-5-type zeolite catalytic substance on which P is supported as the useful hydrocarbon generation catalyst (olefin generation catalyst) filled in the useful hydrocarbon generating step, and setting the reaction temperature to 320°C, GHSV=2000 h⁻¹, the pressure to 5.0 MPa, and the supplied H₂/CO ratio to 2.0.

### (Example 4-2)

Aromatic hydrocarbons (mixture of benzene, toluene, xylene and trimethylbenzene) were produced similarly to the above Examples 1-1 to 1-6, except for using a catalyst made by physically mixing, in a 1:1 weight ratio, a methanol synthesis catalytic substance and a ZSM-5-type zeolite catalytic substance (ZnCr₂O₄/ZSM-5) in which 1 wt% of ZnCr₂O₄ is physically mixed as the useful hydrocarbon generation catalyst (aromatic hydrocarbon generation catalyst) filled in the useful hydrocarbon generating step, and setting the reaction temperature to 350°C, GHSV=500 h⁻¹, the pressure to 5.0 MPa, and the supplied H₂/CO ratio to 2.5.

### (Example 4-3)

Gasoline was produced similarly to the above Examples 1-1 to 1-6, except for using a catalyst made by physically mixing, in a 1:1 weight ratio, a methanol synthesis catalytic substance and a ZSM-5-type zeolite catalytic substance as the useful hydrocarbon generation catalyst (gasoline generation catalyst) filled in the useful hydrocarbon generating step, and setting the reaction temperature to 370°C, GHSV=3700 h⁻¹, the pressure to 5.0 MPa, and the supplied H₂/CO ratio to 2.0.

### (Example 4-4)

Liquid hydrocarbon was produced similarly to the above Examples 1-1 to 1-6, except for using 0.5 wt% Co-supporting SiO₂ (FT synthesis catalyst) as the useful hydrocarbon generation catalyst (liquid hydrocarbon generation catalyst) filled in the useful hydrocarbon generating step, and setting the reaction temperature to 230°C, GHSV=3000 h⁻¹, the pressure to 2.0 MPa, and the supplied H₂/CO ratio to 2.0.

### (Comparative Examples 4-1 to 4-4)

Propylene which is an olefin, aromatic hydrocarbons (mixture of benzene, toluene, xylene and trimethylbenzene), gasoline and liquid hydrocarbon were respectively produced in the useful hydrocarbon generating step similarly to Examples 4-1 to 4-4, except for including the methanation step, the dry reforming step and the useful hydrocarbon generating step, but not including the recycling step, the hydrogen recycling step and the carbon dioxide supplying step.

The generated amounts of the target useful hydrocarbon and carbon dioxide contained in the third gas, and the generated amounts of carbon dioxide and carbon monoxide contained in the second gas were analyzed by the same method as described above, and the CO conversion ratio of the useful hydrocarbon generating step, carbon yield of target useful hydrocarbon, as well as the carbon dioxide discharge amount discharged outside the process system were calculated. The results are shown in Table 5.

**[Table 5]**

| | Useful hydrocarbon | Inlet of dry reforming step | | | Dry reforming | | Useful hydrocarbon generating step | | |
|---|---|---|---|---|---|---|---|---|---|
| | | CO₂ concentra tion (%) | Ratio (M_{HC}/M_{CO2}) (carbon mole ratio) | Lower hydrocarbons / (lower hydrocarbons + C5 or higher hydrocarbons) (carbon mole ratio) | Reaction temperatu re (°C) | CO₂ conversi on rate (%) | CO conversio n rate (%) | Carbon yield of useful hydrocarbon (target substance/process charged CO₂) | CO₂ discharge amount (mol - CO₂/mol - process charged CO₂) |
| Example 4-1 | Propylene | 49 | 1.00 | 1.00 | 800 | 94.8 | 95 | 0.19 | 0.02 |
| Example 4-2 | Aromatic hydrocarbons | 41 | 1.00 | 1.00 | 800 | 94.2 | 60 | 0.12 | 0.02 |
| Example 4-3 | Gasoline | 49 | 1.00 | 1.00 | 800 | 94.8 | 93 | 0.19 | 0.02 |
| Example 4-4 | Liquid hydrocarbon | 42 | 1.00 | 1.00 | 800 | 94.2 | 60 | 0.27 | 0.01 |
| Comparative Example 4-1 | Propylene | no recycling | | | - | - | 95 | 0.11 | 0.17 |
| Comparative Example 4-2 | Aromatic hydrocarbons | | | | - | - | 60 | 0.06 | 0.15 |
| Comparative Example 4-3 | Gasoline | | | | - | - | 93 | 0.11 | 0.15 |
| Comparative Example 4-4 | Liquid hydrocarbon | | | | - | - | 60 | 0.20 | 0.04 |

As shown in Tables 1 to 5, the above-described Examples could stably produce the useful hydrocarbon over a long period of time, while achieving a decrease in carbon dioxide and a suppression of emission of carbon dioxide, due to having the methanation step using carbon dioxide as a main component of the raw materials, the dry reforming step, the useful hydrocarbon generating step and the recycling step of recycling the carbon dioxide within the system. In addition, by including the combusting step after the useful hydrocarbon generating step, the yield of the useful hydrocarbon could be further improved, while carrying out an additional decrease in carbon dioxide and suppression of emission of carbon dioxide. On the other hand, due to not including the recycling step in the above-described comparative examples, in addition to not being able to achieve a decrease in carbon dioxide or a suppression of emission of carbon dioxide, the production of the useful hydrocarbon over a long period of time was poor.

### EXPLANATION OF REFERENCE NUMERALS

S10 methanation step
S20 dry reforming step
S30 useful hydrocarbon generating step
S40 recycling step
S50 hydrogen recycling step
S60 moisture removing step

## Claims

1. A useful hydrocarbon production method comprising:
a methanation step of generating a first gas containing methane from a mixed gas containing hydrogen and carbon dioxide;
a dry reforming step of generating a second gas containing carbon monoxide and hydrogen from the first gas;
a useful hydrocarbon generating step of generating a third gas containing a useful hydrocarbon from the carbon monoxide and hydrogen in the second gas; and
a recycling step of separating a dry reforming recycle gas containing at least carbon dioxide from the third gas, and supplying the dry reforming recycle gas to the dry reforming step.

2. The useful hydrocarbon production method according to claim 1, wherein the recycling step adjusts a supplied amount of the dry reforming recycle gas to be supplied to the dry reforming step, according to a ratio (M_{HC}/M_{CO2}) of a total carbon mole number M_{HC} of hydrocarbons supplied to the dry reforming step relative to a total carbon mole number M_{CO2} of carbon dioxide in the first gas and carbon dioxide in the dry reforming recycle gas supplied to the dry reforming step.

3. The useful hydrocarbon production method according to claim 1, wherein a CO₂ concentration of the dry reforming recycle gas is 40% or more.

4. The useful hydrocarbon production method according to claim 1, further comprising a hydrogen supplying step of supplying hydrogen to the useful hydrocarbon generating step.

5. The useful hydrocarbon production method according to claim 1, further comprising a hydrogen recycling step of separating hydrogen from the third gas and supplying the hydrogen to the useful hydrocarbon generating step.

6. The useful hydrocarbon production method according to claim 5, wherein the hydrogen recycling step adjusts a supplied amount of hydrogen to be supplied to the useful hydrocarbon generating step according to a mole number M_{H} of hydrogen in the useful hydrocarbon generating step.

7. The useful hydrocarbon production method according to claim 1, further comprising a moisture removing step of removing moisture contained in the first gas discharged from the methanation step.

8. The useful hydrocarbon production method according to claim 1, further comprising an adjusting step of adjusting a mole ratio (M_{H}/M_{CO}) of a mole number M_{H} of hydrogen relative to a mole number M_{CO} of carbon monoxide in the second gas obtained in the dry reforming step.

9. The useful hydrocarbon production method according to claim 1, further comprising a combusting step of separating a substance containing carbon atoms from the third gas and combusting the substance containing carbon atoms to generate carbon dioxide,
wherein the recycling step supplies the dry reforming recycle gas and carbon dioxide generated in the combusting step to the dry reforming step.

10. The useful hydrocarbon production method according to any one of claims 1 to **9,** wherein the useful hydrocarbon is at least one type of hydrocarbon selected from the group consisting of olefins, aromatic hydrocarbons, gasoline, liquefied petroleum gas and liquid hydrocarbons.

11. The useful hydrocarbon production method according to any one of claims 1 to 9, wherein the useful hydrocarbon is liquefied petroleum gas.

12. The useful hydrocarbon production method according to any one of claims 1 to 9, wherein the useful hydrocarbon is liquefied petroleum gas, and hydrocarbons other than C3 to C4 hydrocarbons are included in the dry reforming recycle gas.

13. The useful hydrocarbon production method according to any one of claims 1 to 9, wherein the useful hydrocarbon is an olefin, and hydrocarbons other than a target olefin are included in the dry reforming recycle gas.

14. The useful hydrocarbon production method according to claim 13, wherein carbon monoxide is further included in the dry reforming recycle gas.

15. The useful hydrocarbon production method according to any one of claims 1 to 9, wherein the useful hydrocarbon is an aromatic hydrocarbon, and hydrocarbons other than a target aromatic hydrocarbon are included in the dry reforming recycle gas.

16. The useful hydrocarbon production method according to claim 15, wherein carbon monoxide is further included in the dry reforming recycle gas.

17. The useful hydrocarbon production method according to any one of claims 1 to 9, wherein the useful hydrocarbon is gasoline, and hydrocarbons other than gasoline are included in the dry reforming recycle gas.

18. The useful hydrocarbon production method according to claim 17, wherein carbon monoxide is further included in the dry reforming recycle gas.

19. The useful hydrocarbon production method according to any one of claims 1 to 9, wherein the useful hydrocarbon is a liquid hydrocarbon, and C4 or lower hydrocarbons are included in the dry reforming recycle gas.

20. The useful hydrocarbon production method according to claim 19, wherein carbon monoxide is further included in the dry reforming recycle gas.

21. A useful hydrocarbon production device comprising:
a methanation unit that generates a first gas containing methane from a mixed gas containing hydrogen and carbon dioxide;
a dry reforming unit that generates a second gas containing carbon monoxide and hydrogen from the first gas;
a useful hydrocarbon generating unit that generates a third gas containing a useful hydrocarbon from the carbon monoxide and hydrogen in the second gas; and
a recycling unit that separates a dry reforming recycle gas containing at least carbon dioxide from the third gas and supplies the dry reforming recycle gas to the dry reforming unit.
